(19) Europäisches Patentamt
European Patent Office
Office européen des brevets

(11) EP 0 825 164 A2

# (12) EUROPEAN PATENT APPLICATION

(43) Date of publication:
**25.02.1998 Bulletin 1998/09**

(21) Application number: **97304046.2**

(22) Date of filing: **11.06.1997**

(51) Int Cl.[6]: **C07B 61/00**, C07C 231/02, C07C 231/24, C07C 269/04, C07C 269/08, C07C 303/38, C07C 303/44, B01L 3/00, B01J 19/00

(84) Designated Contracting States:
**BE CH DE ES FR GB IT LI NL SE**

(30) Priority: **14.06.1996 US 19792 P**

(71) Applicant: **ELI LILLY AND COMPANY**
**Indianapolis, Indiana 46285 (US)**

(72) Inventors:
- **Kaldor, Stephen Warren**
  **Indianapolis, Indiana 46254 (US)**
- **Fritz, James Erwin**
  **McCordsville, Indiana 46055 (US)**

(74) Representative: **Hudson, Christopher Mark et al**
**Lilly Industries Limited**
**European Patent Operations**
**Erl Wood Manor**
**Windlesham Surrey GU20 6PH (GB)**

## (54) Scavenger assisted combinatorial process for preparing libraries of amides, carbamates and sulfonamides

(57) This invention relates to a novel solution phase process for the preparation of amide, carbamate, and sulfonamide combinatorial libraries. These libraries have utility for drug discovery and are used to form well-plate components of novel assay kits.

EP 0 825 164 A2

## Description

This invention relates to a solution phase synthesis of diverse combinatorial libraries of amides, carbamates, and sulfonamides. These libraries are useful for discovery of lead compounds for drug development.

Research and development expenses account for the largest outlay of capital in the drug industry. Synthesis of compounds is an expensive and time consuming phase of research and development. Historically, research chemists individually synthesized and analyzed hundreds of high purity compounds for biological screening to develop pharmaceutical leads. Although past methods brought new drugs to market, the limitations of individual synthesis and insistence on compound characterization considerably slowed the discovery process.

The need for more rapid and less expensive drug discovery methodology is increasingly important in today's competitive drug industry.

More recently, modern drug discovery has used the methods of combinatorial chemistry to generate large numbers (viz., about $10^2$ to $10^6$) of compounds generically referred to as "libraries." An important objective of combinatorial chemistry is to generate lead compounds for pharmaceutical research.

Theoretically, the total number of compounds which may be produced for a given library is limited only by the number of reagents available to form substituents on the variable positions on the library's molecular scaffold. The combinatorial process lends itself to automation, both in the generation of compounds and their biological screening.

Combinatorial chemistry may be performed in a manner where libraries of compounds are generated as mixtures with complete identification of individual compounds postponed until after positive screening results are obtained. However, a preferred form of combinatorial chemistry is "parallel array synthesis" where individual reaction products (most often individual compounds) are synthesized together, but are retained in separate vessels. For example, the library compounds are held in the individual wells of 96 well microtiter plates. Use of standardized microtiter plates or equivalent apparatus is advantageous because such an apparatus is readily manipulated by programmed robotic machinery.

Conventionally, combinatorial chemistry is conducted on a solid phase support, normally a polymer. The library scaffold is cleavably tethered to the solid support by a chemical linker. Reactions are carried out to modify the scaffold while tethered to the solid support. In a final step, the product is cleaved and released from the solid support. A general procedure for conventional solid phase synthesis is illustrated by the following scheme where the shaded circle symbol is, for example, a high molecular weight polymer:

**Solid-Phase Synthesis:**

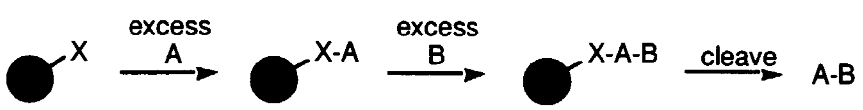


Variations in reagents (e.g., "A", "B", in the general scheme, supra.) produce the desired structural diversity. Separation of solid phase product and unreacted soluble reactant is done by simple separation techniques such as filtration, decantation, or washing. These separation solid phase synthesis techniques have general applicability to a wide variety of combinatorial reactants and lend themselves to large scale simultaneous/automated library creation.

The rate determining step in small molecule synthesis is typically not actual construction of the desired new chemical entities. Rather, the difficulty of synthesis is frequently caused by the task of isolating reaction product from unreacted starting materials, by-products or other impurities.

Unfortunately, it is not always practicable to tether a desired combinatorial scaffold to a solid support. A significant number of combinatorial reaction schemes are desirably done in solution phase. Moreover, not all desired solution phase reactions are driven to completion using near stoichiometric ratios of reactants. Frequently, one reagent is added in considerable excess to drive a solution phase reaction to completion. The result is a reaction medium with soluble product and soluble unreacted co-reactant. Consequently, traditional organic synthetic methods often require complex purification strategies which limit their use, particularly in combinatorial syntheses.

Polymeric reagents have been known and used for general chemical synthesis in various roles; such as follows:

a) The article, *Cyanoborohydride supported anion exchange resin* as *a selective reducing agent* by Hutchins, Robert O.: Natale, Nicholas R. and Taffer, Ira M.; J.C.S. Chem Comm., pg. 1088-9, 1978 describes various reactions including reductive amination using cyanoborohydride anion on ion-exchange resin. The spent resin reagent is removed by simple filtration and washing.

b) The article, *Synthesis and Reactivity of Polymer-Supported Reducing Agents with Chemically Modified Surfaces*

by Menger, Fredric M., Hiraku, Shinozaki, and Lee, Hsueh-Chi; J. Org. Chem 1980, 45, 2724-2725 describes borohydride and cyanoborohydride functional anion exchange resins for carbonyl group reduction. Aldehydes and ketones are reduced to form alcohols by use of polymeric reagents.

c) US Patent No. 3,873,621 describes reductive amination reactions carried out using alkali-metal and quaternary ammonium cyanoborohydrides.

d) The article, *The reduction of α,β- unsaturated nitroalkenes to nitroalkanes with borohydride supported on an ion exchange resin,* by Goudgaon, Naganna, M.; Wadgaonkar, Prakash P.; and Kabalka, George W.; Synthetic Communications, 19(5&6), 805-811 (1989) describes the use of polymer supported borohydride reagent used to reduce nitroalkenes to nitroalkanes.

e) The article, *Borohydride reducing agent derived from anion exchange resin: Selective reduction of α,β-carbonyl compound* by Sande, A.R. et. al., Tetrahedron Letters, Vol. 25, No. 32, pp 3501-3504 1984 describes the use of borohydride exchange resin for the reduction of cyclic and acyclic ketones to alcohols with the attendant advantage of simple separation by filtration to give product free of boron moiety.

f) The article, A *reductive amination/lactamization procedure using borohydride reagents* by Abdel-Magid, Ahmed F.; Harris, Brice D. and Maryanoff, Cynthia A.; Synlett, pgs. 81-3, January 1994 describes reductive amination of carbonyl compounds using sodium triacetoxyborohydride.

g) The article, *New Probes for the Study of Acylation Reactions,* by J. Rebek, D. Brown, and S. Zimmerman (Contribution No. 3481), Journal of the American Chemical Society, 97:15, p.4408, July 23, 1975, JACS 97:15 July 23, 1975 describes the use of polymer bound isocyanate to activate carboxylic acid.

h) The article, *Chemical Modification of Polymers. Borohydride Reducing Agents Derived from Anion Exchange Resins,* by H. W. Bibson and F.C. Bailey, J.C.S. Chem. Comm., p. 815, 1977 describes the preparation of an insoluble polymer bound reducing agent by reacting anion exchange resins of the quaternary ammonium type with aqueous NaBH4.

i) The article, *Solid Phase Synthesis of Oligosaccharides. I. Preparation of the Solid Support. poly(p-(l-propen-3-ol-1-yl) styrene,* by J. M. Frechet and C. Schuerch, Journal of the American Chemical Society, 93:2, p. 492-496 describes the preparation of -CHO functional polymers by reacting chloromethylated resin with methyl sulfoxide and sodium bicarbonate.

j) U.S. Patent No. 3,576,870 describes the purification of dimethylacetamide by removing acetic anhydride with a basic ion exchange resin containing primary or secondary amino groups.

k) The article, *Use of Polymeric Nucleophiles for the Selective Binding and Removal of α-Methylene-γ-butyrolactone Allergens from Complex Mixtures,* by A. Cheminat and C. Benezra, Tetrahedron Letters, Vol. 21, p. 617-619 (1980) describes an amine functional polymer used as a nucleophile for removal of an α,β-unsaturated lactone electrophile.

l) The article, *Polymeric De-blocking Agents for the Fluoren-9-ylmethoxycarbonyl (FMOC) Amino-protecting Group,* by L.S. Carpino and J.R. Williams, J.C.S. Chem. Comm., p.450-451, (1978) describes the use of a resin bound piperazine to remove FMOC with subsequent scavenging of the dibenzofulvene contaminant.

m) The article, *Piperazino-Functionalized Silica Gel as a Deblocking-Scavenging Agent for the* 9-*Fluorenylmethyloxycarbonyl Amino-Protecting Group,* by L.A. Carpino, E.M.E. Mansour, and J. Knapczyk, J. Org. Chem., 48, p. 666-669 (1983) describes a silica bound piperazine to remove FMOC with subsequent scavenging of the dibenzofulvene contaminant.

n) The article, Preparation of High Capacity Aminomethyl-Polystyrene Resin, by C. C. Zikos and N.G. G. Ferderigos, Tetrahedron Letters, Vol. 36, No. 21, p. 3741-3744, 1995, describes the preparation of an amine functional resin.

o) U.S. Patent No. 5,244,582 relates to reactive groups immobilized on inorganic substrates such as glass. Such immobilized groups can be used to remove nitrosating agents in liquids, etc.

There remains a need to develop solution phase combinatorial processes for making libraries.

Summary of the Invention

Combinatorial chemistry may be used at two distinct phases of drug development. In the discovery phase highly diverse libraries are created to find lead compounds. In a second optimization phase, strong lead compounds are much more narrowly modified to find optimal molecular configurations. The method of this invention has particular advantages for making diverse libraries of amide, carbamate, and sulfonamide compounds useful for finding new lead compounds.

FIG. 1 is a top view of a wellplate apparatus.

FIG. 2 is a side view of a wellplate apparatus.

## Detailed Description of the Invention

### I. Definitions:

The following terms have the meaning defined below when used in this specification of the invention:

"Acyl halide" means a compound of the general formula;

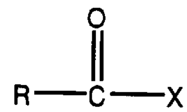

where X is halo and R is an organic group.

"Assay kit" means an assemblage of two cooperative elements, namely, (i) a wellplate apparatus, and (ii) biological assay materials.

"Biological assay materials" are materials necessary to conduct a biological evaluation of the efficacy of any library compound in a screen relevant to a selected disease state.

"Directed Library" is a collection of compounds created by a combinatorial chemistry process for the purpose of optimization of the activity of a lead compound, wherein each library compound has a common scaffold, and the library, considered in its entirety, is a collection of closely related homologues or analogues to the lead compound (compare to "Diverse library").

"Diverse library" means a library where the substituents on the combinatorial library scaffold are highly variable in constituent atoms, molecular weight, and structure and the library, considered in its entirety, is not a collection of closely related homologues or analogues (compare to "Directed library").

"Electrophile" means an electron seeking reagent.

"Lead compound" means a compound in a selected combinatorial library for which the Assay kit has revealed significant activity relevant to a selected disease state.

"Library" is a collection of compounds created by a combinatorial chemical process, said compounds having a common scaffold with one or more variable substituents.

"Library compound" means an individual reaction product (usually a single compound) in a library produced by the method of the invention, either the amide, carbamate, or sulfonamide libraries.

"Parallel array synthesis" means a method of conducting combinatorial chemical synthesis of libraries wherein the individual combinatorial library reaction products are separately prepared and stored without prior or subsequent intentional mixing.

"Reaction zone" means the individual vessel location where the combinatorial chemical library compound preparation process of the invention is carried out and individual library compounds synthesized. Suitable reaction zones are the individual wells of a wellplate apparatus.

"Scaffold" means the invariant region (viz., core) of the compounds which are members of a library (viz., amide, carbamate, or sulfonamide).

"Simultaneous synthesis" means making of library of compounds within one production cycle of a combinatorial method (not making all library compounds at the same instant in time).

"Solid-supported scavenger" means a reaction medium insoluble solid substance containing chemical functionality reactive with the soluble impurity (viz., usually excess reactant) desired to be removed from the reaction medium in the presence of soluble product.

"Substituents" are chemical radicals which are bonded to the scaffold through the combinatorial synthesis process. The different functional groups account for the diversity of molecules throughout the library and are selected to impart diversity of biological activity to the scaffold in the case of diverse libraries, and optimization of a particular biological activity in the case of directed libraries.

"Reagent" means a reactant, any chemical compound used in the combinatorial synthesis to place substituents on the scaffold of a library.

"Wellplate apparatus" means a structure capable of holding a plurality of library compounds in dimensionally fixed and defined positions.

"Non-interfering substituent", refers to a halo or organic (non-hydrogen) radical suitable for substitution as $R_1$, $R_2$, $R_3$ or $R_4$ in the reactants used in the process of making a combinatorial amide, carbamate, or sulfonamide library. Non-interfering substituents are those that do not significantly impede the solution phase processes of the invention or interfere with the use of a solid phase scavenger in said processes. Suitable non-interfering radicals include, but are not limited to, $C_1$-$C_{10}$ alkyl, $C_2$-$C_{10}$ alkenyl, $C_2$-$C_{10}$ alkynyl, $C_1$-$C_{10}$ alkoxy, $C_7$-$C_{12}$ aralkyl, $C_7$-$C_{12}$ alkaryl,

$C_3$-$C_{10}$ cycloalkyl, $C_3$-$C_{10}$ cycloalkenyl, phenyl, substituted phenyl, toluyl, xylenyl, biphenyl, $C_2$-$C_{12}$ alkoxyalkyl, $C_1$-$C_6$ alkylsulfinyl, $C_1$-$C_{10}$ alkylsulfonyl, $-(CH_2)_m$-O-(aryl), $-(CH_2)_m$-O-($C_1$-$C_{10}$ alkyl), aryl, substituted aryl, substituted alkoxy, amidino, fluoroalkyl, aryloxyalkyl, heterocyclic radical, substituted heterocyclic radical, and nitroalkyl; where m is from 1 to 8. Preferred non-interfering radicals are $C_1$-$C_{10}$ alkyl, $C_2$-$C_{10}$ alkenyl, $C_1$-$C_{10}$ alkoxy, $C_7$-$C_{12}$ aralkyl, $C_7$-$C_{12}$ alkaryl, $C_3$-$C_{10}$ cycloalkyl, $C_3$-$C_{10}$ cycloalkenyl, phenyl, $-(CH_2)_m$-O-($C_1$-$C_{10}$ alkyl), aryl, and substituted aryl.

"$C_x$-$C_y$ alkyl" means a straight or branched chain hydrocarbon of between x and y carbon atoms.

"$C_x$-$C_y$ cycloalkyl" means a ring of between x and y carbon atoms having at least one fully saturated bond. "Aryl" means one or more aromatic rings, each of 5 or 6 carbon atoms. Multiple aryl rings may be fused, as in naphthyl, or unfused, as in biphenyl.

"Substituted Aryl" having one or more non-interfering groups as substituents.

"Halo" means chloro, fluoro, iodo or bromo.

"Heterocycle" means one or more rings of 5, 6, or 7 atoms with or without unsaturation or aromatic character and at least one ring atom which is not carbon. Preferred heteroatoms include sulfur, oxygen, and nitrogen. Multiple rings may be fused, as in quinoline or benzofuran.

"Substituted heterocycle" means heterocycle with one or more side chains formed from non-interfering substituents.

II. Amide combinatorial library - General Description:

The amide library of the invention is a combinatorial library formed from (i) primary amine or secondary amine reactants and (ii) acyl halide reactants. Individual amide library compounds are represented by the general formula (I):

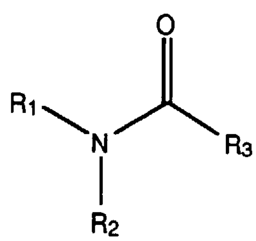

(I)

where $R_1$, $R_2$, and $R_3$ are substituents as defined below in sections III, IV, and V.

A preferred amide library is formed from diverse amine reactants and diverse acyl halide reactants.

The following sections III, IV, and V describe a combinatorial solution phase process for making amide libraries.

III. Amide combinatorial library - Process for making by scavenger assisted solution phase Drocess.

This invention is a scavenger assisted combinatorial process for preparing a library of compounds having an amide scaffold with three variable substituents, said compounds represented by the formula;

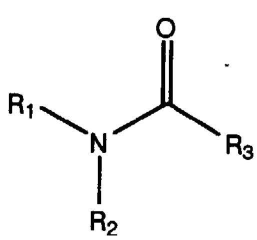

(I)

where $R_1$, $R_2$, and $R_3$ are non-interfering substituents, said process comprising the steps of:

a) adding to each reaction zone at least (n) equivalents of a solvent soluble primary or secondary amine reactant represented by the formula:

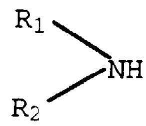

where $R_1$ and $R_2$ are independently selected from hydrogen or non-interfering substituents;
b) adding to each reaction zone of step (a) containing a liquid medium;

(i) at least 1.1(n) equivalents of an acyl halide represented by the formula;

$$R_3C(O)X$$

where X is halo and $R_3$ is a non-interfering substituent, and;
ii) a base in an amount sufficient to neutralize the acid, HX, formed;

c) adding to each reaction zone of step (b) a solid supported amine functional scavenger represented by the formula;

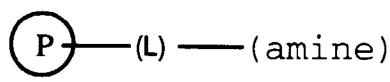

wherein;

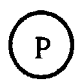

is a solid-support insoluble in the liquid medium of the reaction zone, and -(L)- is a divalent linking group, (amine) is either a primary or secondary amine substituent; and adding said scavenger in an amount at least equal to the excess equivalents of unreacted acyl halide reactant used in step (b), and maintaining said reaction zone at a temperature and for a time sufficient to permit reaction of said excess acyl halide reactant and said scavenger;
d) separating the solid supported scavenger from each reaction zone of step (c) and recovering each substantially purified amide library compound.

The "adding to each reaction zone" requirement for the amine and acyl halide reactant in steps (a) and (b) means that different amines and acyl halides may be added to each reaction zone in the library forming process, if desired. In one embodiment of the process of the invention, each combination of amine and acyl halide added to each library reaction zone (e.g., wells of a wellplate) is different. Thus, the same amine may be added to each row of a wellplate apparatus (as per Fig. 1) and the same aldehyde reactant may be added to the same column of a wellplate apparatus to give a different combination of reactants in each well (viz., reaction zone) that will expectantly yield a different library compound. Alternatively, where it is desirable to have replicate samples, the same combination of amine and acyl halide may be added to different reaction zones.

Detail of Operation for the Amide Librarv Process - Step (a):

The amide library making process is preferably conducted using in step (a) a primary or secondary amine;

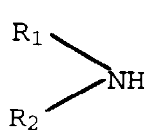
,

wherein $R_1$ and $R_2$ are independently selected from hydrogen (provided that $R_1$ and $R_2$ are not both hydrogen) or a

non-interfering radical selected from $C_1$-$C_{10}$ alkyl, $C_2$-$C_{10}$ alkenyl, $C_2$-$C_{10}$ alkynyl, $C_1$-$C_{10}$ alkoxy, $C_7$-$C_{12}$ aralkyl, $C_7$-$C_{12}$ alkaryl, $C_3$-$C_{10}$ cycloalkyl, $C_3$-$C_{10}$ cycloalkenyl, phenyl, substituted phenyl, toluyl, xylenyl, biphenyl, $C_2$-$C_{12}$ alkoxyalkyl, $C_1$-$C_6$ alkylsulfinyl, $C_1$-$C_{10}$ alkylsulfonyl,-$(CH_2)_m$-O-(aryl), -$(CH_2)_m$-O-($C_1$-$C_{10}$ alkyl), aryl, substituted aryl, substituted alkoxy, amidino, fluoroalkyl, aryloxyalkyl, heterocyclic radical, substituted heterocyclic radical, and nitroalkyl; where m is from 1 to 8. Preferred non-interfering radicals are $C_1$-$C_{10}$ alkyl, $C_2$-$C_{10}$ alkenyl, $C_1$-$C_{10}$ alkoxy, $C_7$-$C_{12}$ aralkyl, $C_7$-$C_{12}$ alkaryl, $C_3$-$C_{10}$ cycloalkyl, $C_3$-$C_{10}$ cycloalkenyl, phenyl, -$(CH_2)_m$-O-($C_1$-$C_{10}$ alkyl), aryl, and substituted aryl.

The amine reactant,

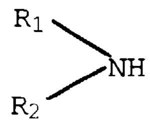

,

is preferably selected from aliphatic, aromatic, and heterocyclic primary amines or secondary amines having a molecular weight of from 50 to 600. Examples of amine reactants suitable for use in the process of the invention are the following:

Primary Amine Reagents --

aniline
cyclopropylamine
cyclobutylamine
(-) -cis-myrtanylamine
cyclopentylamine
cyclohexylamine
2 -methylcyclohexylamine
2,3-dimethylcyclohexylamine
4-methylcyclohexylamine
(aminomethyl)cyclohexane
3-aminomethyl-3,5,5-trimethylcyclohexanol
1,2,3,4-tetrahydro-1-naphthylamine
cyclooctylamine
1-tyrosine methyl ester
2-(2-aminoethyl)-1-methylpyrrolidine
n-(2-aminoethyl)pyrrolidine
n-(3'-aminopropyl)-2-pyrrolidinone
furfurylamine
cyclododecylamine
1-aminoindan
dl-1-(1-naphthyl)ethylamine
1-naphthalenemethylamine
cycloheptylamine
(1s,2s)-(+)-2-amino-1-phenyl-1,3-propanediol
dl-2-amino-3-methyl-1-butanol
l-isoleucinol
l-phenylalaninol
dl-4-chlorophenylalaninol
d-(-)-leucinol
l-methioninol
histamine
tetrahydrofurfurylamine
dl-alpha-methyltryptamine
tryptamine
5-methoxytryptamine
6-methoxytryptamine
piperonylamine

n-(2-aminoethyl)morpholine
n-(3-aminopropyl)morpholine
2-(2-aminoethylamino)-5-nitropyridine
2-(aminomethyl)pyridine
2-(2-aminoethyl)pyridine
3-(aminomethyl)pyridine
4-(aminomethyl)pyridine
ethyl 4-amino-1-piperidinecarboxylate
4-amino-1-benzylpiperidine
1-(2-aminoethyl)piperidine
1-(3-aminopropyl) -2-pipecoline
1,2-diamino-2-methylpropane
benzhydrylamine
d-(-)-alpha-phenylglycinol
1,2-diphenylethylamine
dl-1-phenylethylamine
(-)-norephedrine
1,2-dimethylpropylamine
isopropylamine
2-methoxyisopropylamine
dl-2-amino-1-propanol
ethyl-3-aminobutyrate
1,3-dimethylbutylamine
3 -amino-1-phenylbutane
2-amino-5-diethylaminopentane
1,5-dimethylhexylamine
sec-butylamine
(+/-)-2-amino-1-butanol
3-aminopentane
2-aminopentane
3-aminoheptane
2 -aminoheptane
2 -aminooctane
benzylamine
2-fluorobenzylamine
2 -chlorobenzylamine
2,4-dichlorobenzylamine
2-methoxybenzylamine
2-ethoxybenzylamine
2-methylbenzylamine
3-fluorobenzylamine
3,4-dichlorobenzylamine
3,4-dimethoxybenzylamine
3-(trifluoromethyl)benzylamine
3-methylbenzylamine
4-fluorobenzylamine
4-chlorobenzylamine
4-methoxybenzylamine
4-methylbenzylamine
2,2,2-trifluoroethylamine
2 -amino-1-phenylethanol
1-amino-2-propanol
3 -amino-1,2-propanediol
2,2-diphenylethylamine
beta-methylphenethylamine
isobutylamine
2-methylbutylamine
2-ethylhexylamine

n-decylamine
n-undecylamine
dodecylamine
tridecylamine
1-tetradecylamine
hexadecylamine
octadecylamine
ethylamine
2-(2-aminoethylamino)ethanol
2-methoxyethylamine
2-(2-aminoethoxy)ethanol
ethanolamine
phenethylamine
2-(2-chlorophenyl)ethylamine
2-(2-methoxyphenyl)ethylamine
3 -methoxyphenethylamine
2-(3,4-dimethoxyphenyl)ethylamine
4-bromophenethylamine
2-(4-chlorophenyl)ethylamine
2-(4-methoxyphenyl)ethylamine
tyramine
2-(4-aminophenyl)ethylamine
2-(p-tolyl)ethylamine
taurine
propargylamine
allylamine
3,3-dimethylbutylamine
3,3-diphenylpropylamine
isoamylamine
propylamine
3 -dimethylaminopropylamine
3 -diethylaminopropylamine
3-(di-n-butylamino)propylamine
3 -isopropoxypropylamine
3-ethoxypropylamine
3-amino-1-propanol
3-phenylpropylamine
4-amino-1-butanol
4-phenylbutylamine
n-amylamine
5-amino-1-pentanol
hexylamine
6-amino-1-hexanol
n-heptylamine
n-octylamine
n-nonylamine
dl-2-amino-1-pentanol
dl-2-amino-1-hexanol
1-(3-aminopropyl)imidazole
3,5-bis (trifluoromethyl)benzylamine
2,4-difluorobenzylamine
2,5-difluorobenzylamine
2,6-difluorobenzylamine
3,4-difluorobenzylamine
4-(trifluoromethyl)benzylamine
2-(trifluoromethyl)benzylamine
4-(2-aminoethyl)benzenesulfonamide
n-(4-aminobutyl)-n-ethylisoluminol

n-butylamine
2-(1-cyclohexenyl)ethylamine
3-methoxypropylamine
3,4,5-trimethoxybenzylamine
3-butoxypropylamine
aminomethylcyclopropane
pentadecylamine
4-(2,4-di-tert-amylphenoxy)butylamine
3-chlorobenzylamine
4-fluoro-alpha-methylbenzylamine
(r)-(+)-bornylamine
n,n-di-n-butylethylenediamine
(r)-(-)-1-cyclohexylethylamine
n,n,2,2-tetramethyl-1,3-propanediamine
1-phenylalanine beta-naphthyl-amide
2-(3-chlorophenyl)ethylamine
2 -amino-1,3-propanediol
2-(2-thienyl)ethylamine
2,3-dimethoxybenzylamine
3,5-dimethoxybenzylamine
2,4-dichlorophenethylamine
2,5-dimethoxyphenethylamine
3-fluoro-5-(trifluoromethyl)benzylamine
4-(trifluoromethoxy)benzylamine
l-leucinol
l-leucine-4-nitroanilide
(r)-(+)-1-(1-naphthyl)ethylamine
(s)-(-)-1-(1-naphthyl)ethylamine
l-valinol
d-valinol
d-phenylalaninol
l-(+) -alpha-phenylglycinol
d-(+) -alpha-methylbenzylamine
l(-)-alpha-methylbenzylamine
(1s,2r)-(+)-phenyl-propanolamine
(s)-(+) -2-amino-1-propanol
d-alaninol
(r)-(-)-sec-butylamine
(s)-(+)-sec-butylamine
(s)-(+) -2-amino-1-butanol
(r)-(-) -2-amino-1-butanol
(r)-(-)-1-amino-2-propanol
(s)-(+)-1-amino-2-propanol
(s)-(-)-2-methylbutylamine
(s)-1-cyclohexylethylamine
oleylamine
1-adamantanemethylamine
(1s,2r)-(+) -2-amino-1,2-diphenylethanol
(1r,2s)-(-)-2-amino-1,2-diphenylethanol
s-benzyl-l-cysteinol
2-(2-(aminomethyl)phenylthio)benzyl alcohol
3 -fluorophenethylamine
2-aminobenzylamine
2 -fluorophenethylamine
4-aminobenzylamine
d-glucamine
(+/-) -2,5-dihydro-2,5-dimethoxyfurfurylamine
(s)-(+)-tetrahydrofurfurylamine

4-fluorophenethylamine
(1s,2s)-(+)-thiomicamine
(-) -3,4-dihydroxynorephedrine
(r)-(+)-1-(p-tolyl)ethylamine
(s)-(-)-1-(p-tolyl)ethylamine
(s)-(-)-2-amino-1,1-diphenyl-1-propanol
(+/-)-exo-2-aminonorbornane
(s) -(+)-2-(aminomethyl)pyrrolidine
3-amino-1-propanol vinyl ether
geranylamine
4-(hexadecylamino)benzylamine
(1r,2r,3r,5s)-(-)-isopinocampheylamine
(1s,2s,3s,5r)-(+)-isopinocampheylamine
n1-isopropyldiethylenetriamine
(s)-tert-leucinol
(r)-(-)-tetrahydrofurfurylamine
dehydroabietylamine
2-bromo-4,5-dimethoxyphenethylamine
(1s,2r)-(-)-cis-1-amino-2-indanol
(1r,2s)-(+)-cis-1-amino-2-indanol.

Additional primary amines suitable for the process of the invention are those represented by the following formulae:

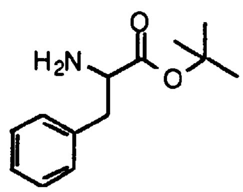
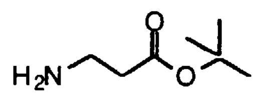

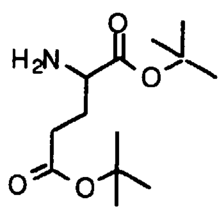
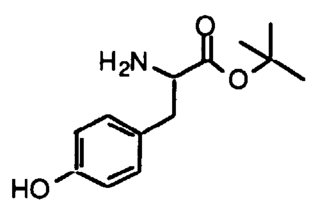

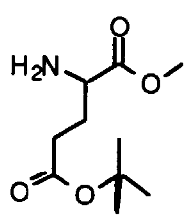
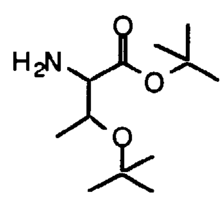

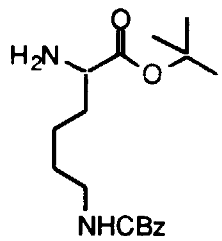

and

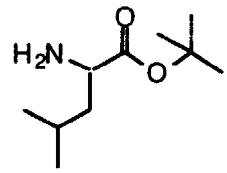

where CBz is Benzyloxycarbonyl.

Secondary Amine Reagents

n-propylcyclopropanemethylamine
(n-butylamino)acetonitrile
n-methyl-beta-alaninenitrile
3-(benzylamino)propionitrile
3,3'-iminodipropionitrile
(r)-(-)-isoproterenol
(1r,2r)-(-)-pseudoephedrine
l-adrenaline
synephrine
2-(methylamino)ethanol
n-benzylethanolamine
2-(ethylamino)ethanol diethanolamine
2-(propylamino)ethanol
heptamethyleneimine
n,n',n"-methylidynetrisformamide
n-isopropylcyclohexylamine
n-methylcyclohexylamine
n-ethylcyclohexylamine
allylcyclohexylamine
diisopropanolamine
n-methyl-d-glucamine
dibenzylamine
noreleagnine
propyleneimine
azetidine
n-omega-acetylhistamine
thiazolidine
3- pyrroline
2,5-dimethyl-3-pyrroline
pyrrolidine
l-prolinamide
l-prolinol
3-pyrrolidinol
n-omega-methyltryptamine
1-piperonylpiperazine
1,2,3, 6-tetrahydropyridine
1-phenylpiperazine
1-(2-methoxyphenyl)piperazine
n-(3-trifluoromethylphenyl)piperazine
1-(4-fluorophenyl)piperazine
1-(4-nitrophenyl)piperazine
4-piperazinoacetophenone
1-ethoxycarbonylpiperazine
1-(4-chlorobenzhydryl)piperazine
n-methylpiperazine

1-benzylpiperazine
1-(pyrrolidinocarbonylmethyl)piperazine
n-isopropyl-l-piperazineacetamide
n-beta-hydroxyethylpiperazine
morpholine
2,6-dimethylmorpholine
thiomorpholine
1,4-dioxa-8-azaspiro[4.5] decane
piperidine
ethyl pipecolinate
2 -methylpiperidine
2 -piperidinemethanol
2-ethylpiperidine
2-piperidineethanol
n,n-diethylnipecotamide
ethyl nipecotate
nipecotamide
3-methylpiperidine
3,3-dimethylpiperidine
3,5-dimethylpiperidine
3 -piperidinemethanol
4-hydroxypiperidine
4-hydroxy-4-phenylpiperidine
4-(4-chlorophenyl) -4-hydroxypiperidine
4-phenylpiperidine
ethyl isonipecotate
4-methylpiperidine
4-benzylpiperidine
1-(2-pyridyl)piperazine
2-(2-methylaminoethyl)pyridine
4-piperidinopiperidine
1-methyl-4-(methylamino)piperidine
decahydroquinoline
1,2,3,4-tetrahydroisoquinoline
hexamethyleneimine
dimethylamine
n-methylbenzylamine
n-methylphenethylamine
n'-benzyl-n,n-dimethylethylenediamine
methylaminoacetaldehyde dimethylacetal
n-methylpropargylamine
dipropargylamine
n-methylallylamine
diallylamine
diisopropylamine
n-isopropylbenzylamine
diisobutylamine
n-methyloctadecylamine
n-ethylmethylamine
n-ethylbenzylamine
diethylamine
n,n-dimethyl-n'-ethylethylenediamine
n,n-diethyl-n'-methylethylenediamine
n,n,n'-triethylethylenediamine
n-benzylglycine ethyl ester
di-sec-butylamine
methyl-n- propylamine
dipropylamine

n-methylbutylamine
n-butylbenzylamine
n-ethyl-n-butylamine
dibutylamine
di(2-ethylhexyl)amine
dipentylamine
di-n-hexylamine
di-n-octylamine
n-benzyl-2-phenylethylamine
9-(methylaminomethyl)anthracene
(s)-(+)-2-(methoxymethyl)pyrrolidine
2 -methylaminomethyl-1,3-dioxolane
pindolol
n-ethylmethallylamine
dicyclohexylamine
1,4,5, 6-tetrahydropyrimidine
n-(trimethylsilylmethyl)benzylamine
4,4-dimethyl-2-imidazoline
(s)-(+)-1-(2-pyrrolidinylmethyl)pyrrolid
n,n,n'-trimethylethylenediamine
n,n,n'-trimethyl-1,3-propanediamine
tetramethylimino-bis-propylamine
(r)-(+)-n-benzyl-1-phenylethylamine
n-ethylisopropylamine
(s)-(+)-2-(anilinomethyl)pyrrolidine
(+/-)-nornicotine
2-(butylamino)ethanol
4-(ethylaminomethyl)pyridine
bis(2-methoxyethyl)amine
4-(1-pyrrolidinyl)piperidine
isonipecotamide
methylisopropylamine
n-methylhexylamine
(r)-(+)-n-methyl-1-phenylethylamine
3-(3-pyridylmethylamino)propionitrile
di-n-decylamine
1-acetylpiperazine
n-methylhomopiperazine
1-ethylpiperazine
dl-adrenaline
trans-1-cinnamylpiperazine
(+)-pseudoephedrine
(-)-ephedrine
d-prolinol
2,6-dimethylpiperidine
(s)-(-)-n-benzyl-1-phenylethylamine
1,3,3-trimethyl-6-azabicyclo(3.2.1)octane
4-(4-bromophenyl) -4-piperidinol
(s)-(-)-n-methyl-1-phenylethylamine
n-methylhomoveratrylamine
(r)-(+)-atenolol
(s)-(-)-atenolol
1-hydroxyethylethoxypiperazine
demecolcine
n-allylcyclopentylamine
mitomycin c
di-beta-d-xylopyranosylamine
cytisine.

Other suitable secondary amines for use in the process of the invention are selected from the group represented by the formula:

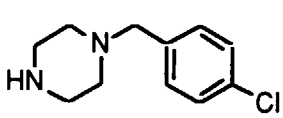
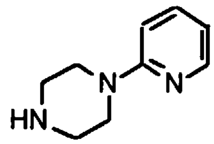
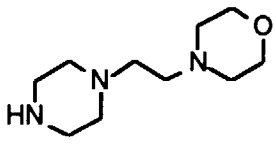
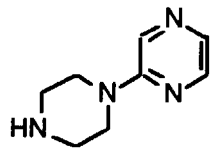
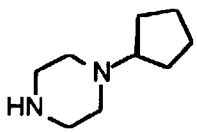
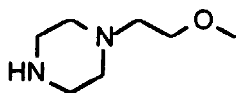
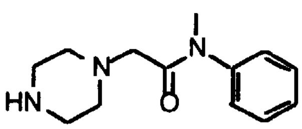
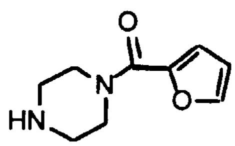
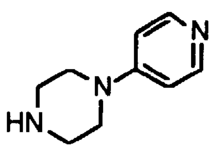
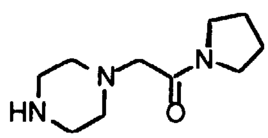
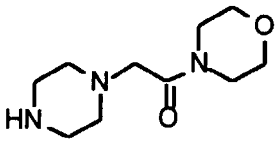
and
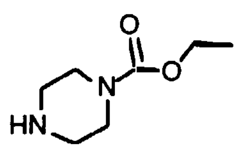
.

<u>Detail of Operation for the Amide Library Process - Step (b)</u>:

The acyl halide reactant is an acyl halide of the formula,

$$R_3C(O)X$$

where X is halo and $R_3$ is a non-interfering substituent selected from $C_1$-$C_{10}$ alkyl, $C_2$-$C_{10}$ alkenyl, $C_2$-$C_{10}$ alkynyl, $C_7$-$C_{12}$ aralkyl, $C_7$-$C_{12}$ alkaryl, $C_3$-$C_{10}$ cycloalkyl, $C_3$-$C_{10}$ cycloalkenyl, phenyl, substituted phenyl, toluyl, xylenyl, biphenyl, $C_2$-$C_{12}$ alkoxyalkyl, $C_1$-$C_6$ alkylsulfinyl, $C_1$-$C_{10}$ alkylsulfonyl, $-(CH_2)_m$-O-(aryl), $-(CH_2)_m$-O-($C_1$-$C_{10}$ alkyl), aryl, substituted aryl, amidino, fluoroalkyl, aryloxyalkyl, heterocyclic radical, substituted heterocyclic radical, and nitroalkyl; where

m is from 1 to 8. Preferred non-interfering radicals are $C_1$-$C_{10}$ alkyl, $C_2$-$C_{10}$ alkenyl, $C_7$-$C_{12}$ aralkyl, $C_7$-$C_{12}$ alkaryl, $C_3$-$C_{10}$ cycloalkyl, $C_3$-$C_{10}$ cycloalkenyl, phenyl, aryl, and substituted aryl.

Preferred acyl halides are aliphatic, aromatic and heterocyclic acyl halides havng a molecular weight from 78 to 600.

Suitable acyl halides useful for making amide libraries by the process of the invention are as follows:

Acyl Halides

3,5-bis (trifluoromethyl)benzoyl chloride
benzoyl chloride
2-bromobenzoyl chloride
2-fluorobenzoyl chloride
pentafluorobenzoyl chloride
2,4-difluorobenzoyl chloride
2,6-difluorobenzoyl chloride
2-chlorobenzoyl chloride
2,4-dichlorobenzoyl chloride
2,6-dichlorobenzoyl chloride
o-acetylsalicyloyl chloride
2-methoxybenzoyl chloride
2,6-dimethoxybenzoyl chloride
2-(trifluoromethyl)benzoyl chloride
o-toluoyl chloride
3-bromobenzoyl chloride
3-fluorobenzoyl chloride
3-chlorobenzoyl chloride
3,4-dichlorobenzoyl chloride
m-anisoyl chloride
3,4-dimethoxybenzoyl chloride
3,4,5-trimethoxybenzoyl chloride
3,5-dimethoxybenzoyl chloride
3-ethoxybenzoyl chloride
isophthaloyl chloride
trimesoyl chloride
3-(trifluoromethyl)benzoyl chloride
m-toluoyl chloride
3-(chloromethyl) benzoyl chloride
4-bromobenzoyl chloride
4-fluorobenzoyl chloride
4-chlorobenzoyl chloride
p-anisoyl chloride
4-ethoxybenzoyl chloride
4-n-butoxybenzoyl chloride
4-n-hexyloxybenzoyl chloride
4-heptyloxybenzoyl chloride
4-biphenylcarbonyl chloride
terephthaloyl chloride
4-(trifluoromethyl)benzoyl chloride
4-tert-butylbenzoyl chloride
p-toluoyl chloride
4-ethylbenzoyl chloride
4-n-propylbenzoyl chloride
4-butylbenzoyl chloride
4-pentylbenzoyl chloride
4-hexylbenzoyl chloride
4-n-heptylbenzoyl chloride
methyl oxalyl chloride
ethyl oxalyl chloride
heptafluorobutyryl chloride
2-acetoxyisobutyryl chloride

pivaloyl chloride
3-chloropivaloyl chloride
2-bromopropionyl chloride
2,3-dibromopropionyl chloride
2,3-dichloropropionyl chloride
o-acetylmandelic acid chloride
itaconyl chloride
methacryloyl chloride
isobutyryl chloride
2-ethylhexanoyl chloride
acetyl chloride
bromoacetyl chloride
chloroacetyl chloride
phenoxyacetyl chloride
4-chlorophenoxyacetyl chloride
methoxyacetyl chloride
phenylacetyl chloride
3,3-dimethylacryloyl chloride
cinnamoyl chloride
fumaryl chloride
ethyl malonyl chloride
tert-butylacetyl chloride
isovaleryl chloride
undecanoyl chloride
lauroyl chloride
myristoyl chloride
palmitoyl chloride
heptadecanoyl chloride
stearoyl chloride
propionyl chloride
3-bromopropionyl chloride
3-chloropropionyl chloride
hydrocinnamoyl chloride
succinyl chloride
3-carbomethoxypropionyl chloride
ethyl succinyl chloride
butyryl chloride
4-bromobutyryl chloride
4-chlorobutyryl chloride
valeryl chloride
5-chlorovaleryl chloride
adipoyl chloride
hexanoyl chloride
6-bromohexanoyl chloride
pimeloyl chloride
heptanoyl chloride
suberoyl chloride
octanoyl chloride
10-undecenoyl chloride
2 -chloro-2,2-diphenylacetyl chloride
dichloroacetyl chloride
alpha-chlorophenylacetyl chloride
2-chloropropionyl chloride
2-iodobenzoyl chloride
4-iodobenzoyl chloride
cyclopropanecarbonyl chloride
trans-2-phenyl-1-cyclopropanecarbonyl chloride
cyclobutanecarbonyl chloride

cyclopentanecarbonyl chloride
3-cyclopentylpropionyl chloride
cyclohexanecarbonyl chloride
4-cyanobenzoyl chloride
2-furoyl chloride
1-naphthoyl chloride
2-naphthoyl chloride
thiophene-2-carbonyl chloride
2-thiopheneacetyl chloride
trimellitic anhydride chloride
2,6-pyridinedicarboxylic acid chloride
2-quinoxaloyl chloride
2-nitrobenzoyl chloride
3-nitrobenzoyl chloride
3,5-dinitrobenzoyl chloride
4-nitrobenzoyl chloride
3,4-dimethoxyphenylacetyl chloride
3-methyladipoyl chloride
3,5-dichlorobenzoyl chloride
2,5-difluorobenzoyl chloride
3,4-difluorobenzoyl chloride
9-fluorenone-4-carbonyl chloride
3,5-difluorobenzoyl chloride
(s)-(-) -n-(trifluoroacetyl)prolyl chloride
benzyloxyacetyl chloride
acetoxy acetyl chloride
3-cyanobenzoyl chloride
2,5-dimethoxyphenylacetyl chloride
3-methoxyphenylacetyl chloride
iminodibenzyl-5-carbonyl chloride
2,4,6-trimethylbenzoyl chloride
tetrafluorosuccinyl chloride
perfluorooctanoyl chloride
diphenylacetyl chloride
alpha-methyl valeroyl chloride
methyl malonyl chloride
ethyl glutaryl chloride
5-bromovaleryl chloride
methyl adipyl chloride
3-cyclohexenecarbonyl chloride
3-isocyanato benzoyl chloride
2,4,6-triisopropylbenzoyl chloride
fluoroacetyl chloride
2-ethoxybenzoyl chloride
piperonyloyl chloride
2,4-dimethoxybenzoyl chloride
2,3,5,6-tetrachloroterephthaloyl chloride
5- (dimethylsulfamoyl)-2-methoxybenzoyl chloride
2-(4-chlorobenzoyl)benzoyl chloride
2,2-bis (chloromethyl)propionyl chloride
cinnamylidenemalonyl chloride
2-phenoxypropionyl chloride
2-phenylbutyryl chloride
2-ethylbutyryl chloride
p-tolylacetyl chloride
gamma-methylvaleroyl chloride
3,3-dichloropivaloyl chloride
1-methyl-1-cyclohexanecarboxylic acid chloride

2-(2,4,5-trichlorophenoxy)acetyl chloride
4-chloro-3-nitrobenzoyl chloride
4-methyl-3-nitrobenzoyl chloride
2,3-dichlorobenzoyl chloride
morpholine-4-carbonyl chloride
p-chlorophenylacetyl chloride
bicyclo[2.2.1] heptane-2-carbonyl chloride
d(-)-alpha-formyloxy-alpha-phenylacetyl chloric
d(-)-alpha-phenylglycine chloride hydrochloride
trifluoroacetyl chloride
pentafluoropropionyl chloride
hexafluoroglutaryl chloride
2-chlorocinnamoyl chloride
o-methoxycinnamyl chloride
5-nitro-2-furoyl chloride
2-chlorobutyryl chloride
4-phenylazobenzoyl chloride
4-n-amyloxybenzoyl chloride
4-decylbenzoyl chloride
4-octylbenzoyl chloride
dl-2-methylbutyryl chloride
linolenoyl chloride
linolelaidoyl chloride
11h-eicosafluoroundecanoyl chloride
9h-hexadecafluorononanoyl chloride
2,3-difluorobenzoyl chloride
2-(benzoyloxymethyl)benzoyl chloride
2,2-dimethylvaleroyl chloride
3,5,5-trimethylhexanoyl chloride
phenothiazine-10-carbonyl chloride
3,4-dimethyl benzoyl chloride
(+) -p-(2-methylbutyl)benzoyl chloride
2,4-dichlorophenoxyacetic chloride
pentadecanoyl chloride
nonadecanoyl chloride
neoheptanoyl chloride
9-anthracenecarbonyl chloride
2-ethoxy-1-naphthoyl chloride
pyrrolidine carbonyl chloride
m-(chlorosulfonyl)benzoyl chloride
2-n-propyl-n-valeroyl chloride
2-chloro-4-nitrobenzoyl chloride
2-phenoxybutyryl chloride
2-chloronicotinyl chloride
6-chloronicotinyl chloride
4-(trifluoromethoxy)benzoyl chloride
2-(trifluoromethoxy)benzoyl chloride
2,6-dichloropyridine-4-carbonyl chloride
3-chlorobenzo[b]thiophene-2-carbonyl chloride
4-chloromethylbenzoyl chloride
neodecanoyl chloride
(phenylthio)acetyl chloride
4-carbethoxyhexafluorobutyryl chloride
octafluoroadipoyl chloride
2-diazo-3,3,3-trifluoropropionylchloride
2-bromobutyryl chloride
arachidoyl chloride
cis-vaccenoyl chloride

11-eicosenoyl chloride
behenoyl chloride
petroselinoyl chloride
palmitoleoyl chloride
tridecanoyl chloride
2-chloro-5-nitrobenzoyl chloride
3-methylthiopropionyl chloride
methyl 4-chlorocarbonylbenzoate
anthraquinone-2-carbonyl chloride
carbazole-n-carbonyl chloride
2-nitrophenoxyacetyl chloride
2-bromo-2-methylpropionyl chloride
2-fluoro-3-(trifluoromethyl)benzoyl chloride
2-fluoro-4-(trifluoromethyl)benzoyl chloride
2-fluoro-5-(trifluoromethyl)benzoyl chloride
3-fluoro-5-(trifluoromethyl)benzoyl chloride
4-fluoro-2-(trifluoromethyl)benzoyl chloride
4-fluoro-3-(trifluoromethyl)benzoyl chloride
2-fluoro-6-(trifluoromethyl) benzoyl chloride
2,3,6-trifluorobenzoyl chloride
2,4,5-trifluorobenzoyl chloride
2,4-di(trifluoromethyl)benzoyl chloride
2,6-di (trifluoromethyl)benzoyl chloride
3-(trifluoromethoxy)benzoyl chloride
m-(fluorosulfonyl)benzoyl chloride
trans-1,2-cyclobutanedicarboxylic acid chloride
3-cyclohexylpropionyl chloride
4-ethyl-2,3-dioxo-1-piperazinecarbonylchloride
isoxazole-5-carbonyl chloride
bromodifluoroacetyl chloride
erucoyl chloride
2,4,6-trifluorobenzoyl chloride
dichlorochrysanthemic acid chloride
isononanoyl chloride
1-adamantanecarbonyl chloride
2,5-bis(trifluoromethyl)benzoyl chloride
2,3,4-trifluorobenzoyl chloride
2,3,4,5-tetrafluorobenzoyl chloride
2,4,6-trichlorobenzoyl chloride
2,4-dichloro-5-fluorobenzoyl chloride
4-methoxyphenylacetyl chloride
trans-3-(trifluoromethyl)cinnamoyl chloride
3-(dichloromethyl) benzoyl chloride
4-isocyanato benzoyl chloride
heneicosanoyl chloride
2-chloroisobutyryl chloride
trans-4-nitrocinnamoyl chloride
3,4,5-trifluorobenzoyl chloride
5-fluoro-2-(trifluoromethyl)benzoyl chloride
2,3,5-trifluorobenzoyl chloride
2-chloro-4-fluorobenzoyl chloride
(-)-alpha-chlorophenylacetyl chloride
2-(para-tolylsulfonyl)acetyl chloride
4-methyl-4-nitrohexanoyl chloride
1-chloro-4-fluorosulfonyl-2-naphthoyl chloride
2,3-dibromo-3-phenylpropionyl chloride
2-menthoxyacetyl chloride
2-phenyl-2-(phenylsulfonyl)acetyl chloride

4,4,4-trifluorocrotonyl chloride
4,4,4-trifluorobutyryl chloride
3,4-dichloro-2,5-thiophenedicarbonyl chloride
pentachlorobenzoyl chloride
4,4,7,7-tetranitrosebacoyl chloride
alpha,alpha'-dimethylsuccinyl chloride
alpha-bromoisovaleryl chloride
benzoyl chloride
oleoyl chloride
methyl suberyl chloride
gamma-linolenoyl chloride
(-)-camphanic acid chloride
4,4'-stilbenedicarbonyl chloride
chlorinated benzoyl chloride
(1r)-(+) -camphanic chloride
2-(4-nitrophenoxy)tetradecanoyl chloride
7-[(chlorocarbonyl)methoxy] -4-methylcoumarin
n,n-bis (2-chloroethyl)carbamoyl chloride
(s)-(-) -2-acetoxypropionyl chloride
linoleoyl chloride
3-chlorotetrafluoropropionyl chloride
3,4-dichloropentafluorobutyryl chloride
7h-dodecafluoroheptanoyl chloride
5h-octafluoropentanoyl chloride
perfluorononanoyl chloride
3h-tetrafluoropropionyl chloride
2-bromo-2,3,3,3-tetrafluoropropanoyl chloride
arachidonoyl chloride
pentachloropropionyl chloride
4-decenoyl chloride
tridecafluoroheptanoyl chloride
undecafluorocyclohexanecarbonyl chloride
4-n-nonylbenzoyl chloride
3-(trichlorogermyl)propionylchloride
3,4,5-triiodobenzoyl chloride
2-(phenylthio)propionyl chloride
2,2,2-triphenylacetyl chloride
d(-)-alpha-azido-phenyl acetyl chloride
4-azido-benzoyl chloride
difluoroacetyl chloride
5-chloropyrazine-2-carbonyl chloride
n-(l-naphthalenesulfonyl)-l-phenylalanyl chloride
n-(4-nitrophenylsulfonyl)-l-phenylalanyl chloride
n-(p-toluenesulfonyl)-l-phenylalanyl chloride
dimethylmalonyl chloride
methyl sebacoyl chloride
2,5-dichloropyridine-3-carbonyl chloride
3-(2,5 xylyloxy) propionyl chloride.

Illustrative acyl chorides suitable for use in the process of the invention are represented by the following formulae:

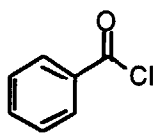

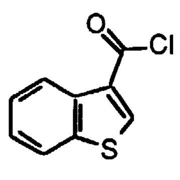

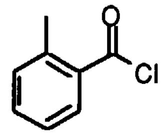
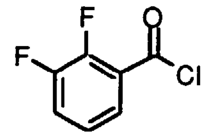
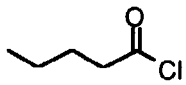
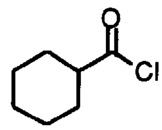
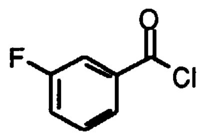
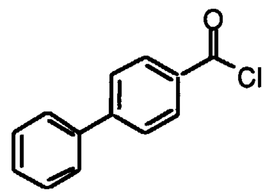
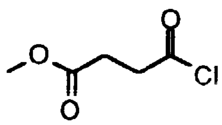
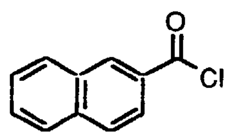
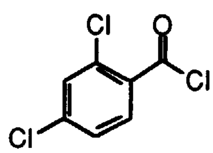
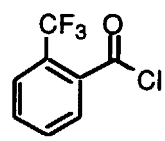

In step (b) a second reagent addition (ii) of neutralizing agent is used to neutralize the acid, HX, resulting from the reaction of the acyl halide and the amine reactant of step (a). Conventional neutralizing agents such as alkali and alkaline-earth hydroxides may be used. However, it is preferred to use a resin bound base such as polyvinyl pyridine as a solid phase scavenger of HX formed in the course of the reaction. Resin bound bases (or other solid neutralizing agents) may be conveniently removed from the reaction in the separation step (d), infra. The amount of acid, HX, anticipated to be formed may be determined by reference to Scheme 1, Step 1, infra, namely, 1 mole of HX formed per mole of amine reagent that reacts with acylhalide.

Detail of Operation for the amide library Process - Step (c):

The solid-supported scavenger is used in the amide library forming process of the invention to remove excess soluble amine reactive reagent. The solid-supported scavenger is represented by the formula:

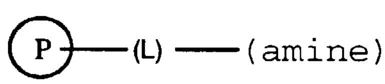

where the;

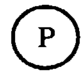

symbol is a solid-support insoluble in the selected reaction medium used in the solution phase amide library making process. Examples of organic solid supports are polymers such as polystyrene divinylbenzene copolymer, polyacrylamide, cellulose and polystyrene. Examples of inorganic solid supports are silica gel, alumina, and controlled pore glass.

The "(amine)" substituent of the scavenger is either a primary or secondary amine substituent, for example;

$-NH_2$,

$-(CH_2)-NH_2$ ,

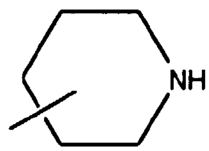 ,

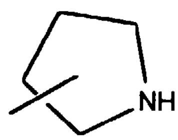 ,

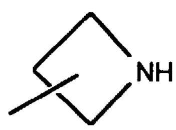 .

The group, -(L)-, is a linking group between the amine radical and the solid support and may be selected from a bond, or any divalent group. Useful linking groups are selected from the following: (bond),

$- O - (CH_2)_x$

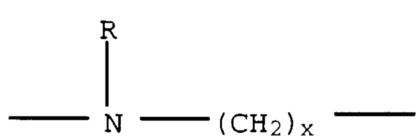

$CH_2 (CH_2)_x -$

where R is hydrogen or $C_1$-$C_{10}$ alkyl and x is zero or from 2 to 10.

The primary amine or secondary amine substituent on the solid supported scavenger readily reacts with excess amine reactive reagent in the library forming process taught herein to covalently bind said excess reagent to the solid

support and permit its simple removal as a solid phase. The effective available amine content of the solid supported scavenger may be readily determined by conventional chemical analysis techniques.

Detail of Operation for the amide library Process - Step (d):

The final step in the amide library forming process of the invention is purification of the library compounds by separating the reacted and unreacted solid supported scavenger from the reaction medium of step (c) and recovering a solution of each substantially purified amide library compound.

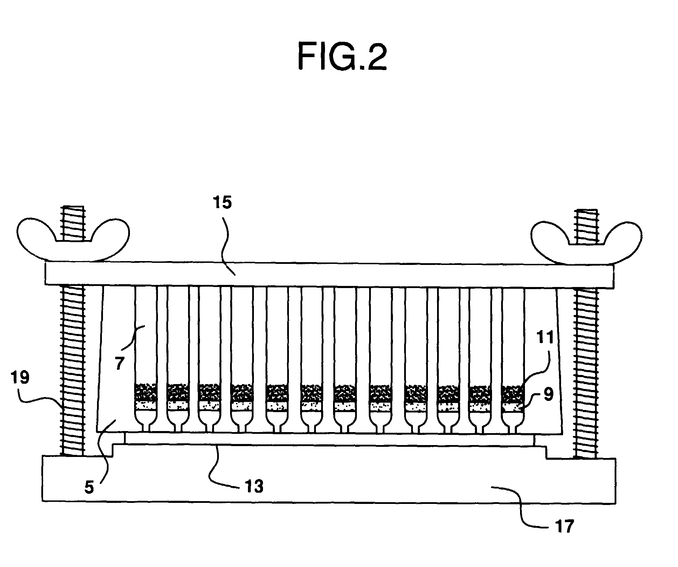

The separation of the solid supported scavenger from the library compound dissolved in the solvent phase of the reaction may be done by any conventional chemical or physical method. Preferred are physical methods which are applicable to all members of a diverse library. Such methods include; (i) filtration, (ii) centrifugation, (iii) decantation, and (iv) washing. Filtration is a particularly preferred form of purification. It is practiced by transporting each solution of library compound through a filter medium which retains the scavenger and transfers the solution phase into a separate vessel. An apparatus using filtration is depicted in Fig. 2, infra.

The purification last step of the process may optionally be supplemented by a solvent removal step in which the solute library compound is removed from its solvent by conventional processes known in the art; such as evaporation, distillation, freeze drying, salting out, solvent extraction, etc.

Other Details of the Amide Library making Process:

Reaction Medium - The reaction medium may be any liquid which has the following characteristics:

(1) the primary or secondary amine and acyl halide reactants are capable of forming a reaction product which is substantially soluble in the reaction medium;
and
(2) the solid supported scavenger, both in unreacted and reacted form, is substantially insoluble in the reaction medium.

Typical reaction media useful in the processes of the invention are toluene, chloroform, methylene chloride, tetrahydrofuran, and acetonitrile.

The Reaction Zone - the process of the invention may be carried out in any vessel capable of holding the liquid reaction medium and having inlet and outlet means.
Preferably the process of the invention is carried out in containers adaptable to parallel array syntheses. Most preferably, the amide library is formed in standard wellplates, such as the 96 well wellplate illustrated in Fig, 1 and/or the wellplate apparatus illustrated in Fig. 2. Each well may be filled by multiple and/or automated apparatus, any of which may be either manually or computer controlled.

The diverse amide library of this invention may take the form of a plurality of wellplates, each wellplate having wells containing a separate reaction product (library compound). In such cases, the library compounds are conveniently identified by their wellplate number and "x" column and "y" wellplate row coordinates.

A preferred technique for practicing the process of the invention is parallel array synthesis. With parallel array synthesis individual reaction products are prepared in each of multiple reaction zones. The amount of amine and acyl halide reactants introduced into each reaction zone will depend on the desired amount of each library compound that is needed for conducting biological assays, archival storage and other related needs. Typically, the desired amount of library compound is from 1 microgram to 50 milligrams.

Proportions of reactants, reaction conditions:

The amount of amine reactant in each reaction zone is represented by the symbol "(n)", where (n) represents the equivalents of the primary or secondary amine reactant put into a reaction zone.

In the diverse amide library making process described herein the acyl halide reactant is the reactant used in excess. The amount of acyl halide used to insure an excess is defined as at least 1.1(n) and preferably a larger excess in the range of from 1.25(n) to 5(n), where the variable (n) is as previously defined. The stoichiometry of the reaction and calculation of equivalent weights of reagents may be done by reference to Scheme 1, infra. The 1.1 multiplier is used to insure at least a 10% stoichiometric excess of the acyl halide to drive the reaction to completion, thereby removing the amine reactant from each reaction zone used to create the amide library. Thus, for example, if 1.25(n) - a 25% mole excess - of the acyl halide reactant is desired, then 107 mg. of benzylamine would be used in step (a) of the process and 176 mg. of benzoyl chloride would be used in step (b) of the process.

Base is added to the reaction zone containing the library compound forming reagents. Preferably a solid supported

base is added in an amount sufficient to neutralize the acid, HX, formed by the reaction.

The reaction zone is maintained at a temperature and for a time sufficient to permit reaction of the amine and acyl halide amine reactants, that is, to complete consumption of the amine and form an amount of amide library compound necessary to conduct biological assays to determine the efficacy of the prepared library compounds.

The time, temperature, and pressure of the combinatorial reaction zones used for the creation of library compounds are not critical aspects of the invention. Reaction times for a single step of the reaction are generally from 0.1 seconds to 24 hours, with times of 1 second to 10 hours being most often used. The temperature of the reaction may be any temperature between the freezing point and the boiling point of the liquid reaction medium, but is generally between -10°C and +60°C, with 10°C to 40°C being preferred and near ambient temperatures (about 20°C-30°C) being most preferred. The reactions may be conducted at subatmospheric pressure or superatmospheric pressure (viz., 60Kg./$m^2$ - 21000 Kg./$m^2$ absolute), but ambient atmospheric pressure (about 10330 Kg./$m^2$, absolute) is most often used.

Endpoint determination - The completion of the reaction between the amide and acyl halide reactant may be determined by a number of conventional techniques. One method is to use thin layer chromatography to determine if the amine reactant is substantially removed from the reaction zones.

Sequence of Operation - The addition of the acyl halide, amine reactants and base to the reaction zone may take place in any order. For example, the amine reactant may be initially added to the reaction zone followed by addition of the acyl halide reactant, or vice versa.
Alternatively, the amine and acyl halide reactants may be simultaneously charged to each reaction zone.

The reaction zone is maintained at a temperature for a time sufficient to permit reaction of said excess acyl halide reactant and said scavenger. Typically, the reaction requires only minutes but the selection of reaction conditions that may be used is the same as set out in the preceding section.

Amide Library Process - Reaction Scheme:

An illustrative reaction scheme for the formation of amide libraries is given in Scheme 1, as follows:

Scheme 1

USE OF AMINOMETHYLATED POLYMER FOR MAKING AMIDE COMBINATORIAL LIBRARIES

STEP 1 - Synthesis of Amide

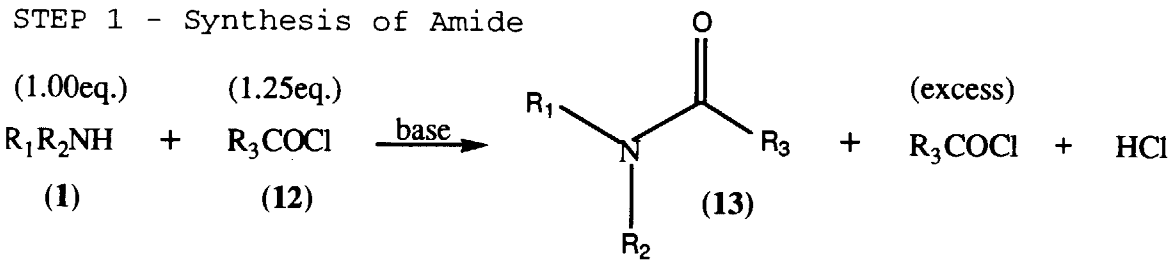


(1) (12) (13)

STEP 2 - Removal of excess $R_3COCl$

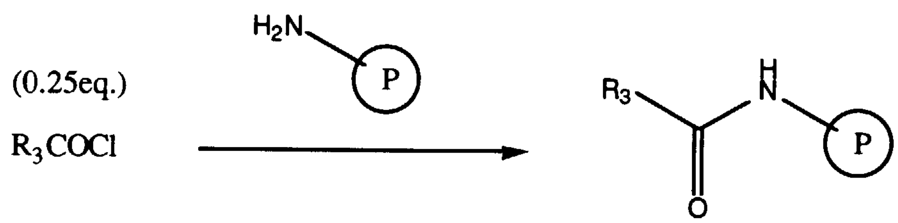


(4) (15)

Preparation of Amides:

In Step 1 a primary or secondary amine (1) is combined in a non-nucleophilic solvent such as ethanol-free chloroform with an excess of acyl chloride (12) and a stoichiometric excess of neutralizing agent (viz., solid supported

dimethylaminopyridine or piperidine-N-polystyrene to neutralize HCl released in the reaction) to form an amide product (13) contaminated with acyl chloride.

In Step 2 there is added to the reaction product of Step 1 a stoichiometric excess of solid supported scavenger (4) in the form of aminomethylated polymer (aminomethylated polystyrene, available from Aldrich Chemical Co.). This scavenger resin-bound primary amine reacts to form a covalent bond between the resin bound amine functionality and the acyl chloride, yielding a resin bound amide (15).

In a final step (not illustrated) the product amide and scavenger in chloroform medium are filtered through a cotton plug. The resin retained by the plug is rinsed free of product by washing with additional chloroform.

A purified amide product is retained (in solution) and may be isolated free of reaction medium by solvent evaporation.

IV. Carbamate combinatorial library - General Description:

The carbamate library of the invention is a combinatorial library formed from (i) primary amine or secondary amine reactant and (ii) organohaloformate reactant. Individual carbamate library compounds are represented by the general formula (V):

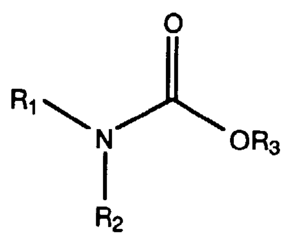

(V)

where $R_1$, $R_2$, and $R_3$ are substituents defined below in sections V, VI, and VII.

A preferred carbamate library is formed from diverse amine reactants and diverse organohaloformate reactants. The following sections V, VI and VII describe a combinatorial solution phase process for making carbamate libraries.

V. Carbamate combinatorial library - Process for making by scavenger assisted solution phase process.

This invention is a scavenger assisted combinatorial process for preparing a library of compounds having a carbamate scaffold with three variable substituents, said compounds represented by the formula (V);

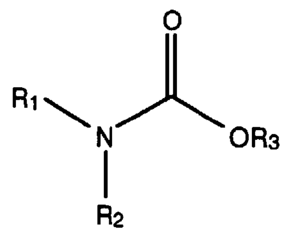

(V)

said process comprising the steps of:

a) adding to each reaction zone at least (n) equivalents of a solvent soluble primary or secondary amine reactant represented by the formula:

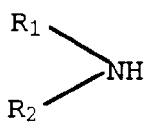

where $R_1$ and $R_2$ are independently selected from hydrogen and non-interfering substituents, provided that $R_1$ and

$R_2$ are not both hydrogen;

b) adding to each reaction zone of step (a) containing a liquid medium;

(i) at least 1.1(n) equivalents of an organohaloformate, represented by the formula,

$$R_3OC(O)X$$

where X is halogen and $R_3$ is a non-interfering substituent; or,

(ii) at least 1.1(n) equivalents of an organocyanoformate, represented by the formula,

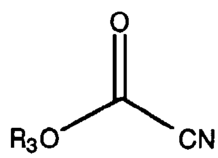

where $R_3$ is a non-interfering substituent; and

(iii) adding to each reaction zone a base in an amount sufficient to neutralize the acid, HX, formed;

c) adding to each reaction zone of step (b) a solid supported amine functional scavenger represented by the formula;

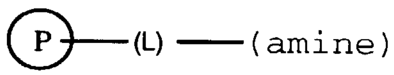

wherein;

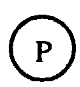

is a solid-support insoluble in the liquid medium of the reaction zone, and -(L)- is a divalent linking group, (amine) is either a primary or secondary amine substituent; and adding said scavenger in an amount at least equal to the excess equivalents of unreacted organohaloformate reactant used in step (b), and maintaining said reaction zone at a temperature and for a time sufficient to permit reaction of said excess organohaloformate reactant and said scavenger;

d) separating the solid supported scavenger from each reaction zone of step (c) and recovering each substantially purified carbamate library compound.

The "adding to each reaction zone" requirement for the amine and either organohaloformate or organochloroformate reactant in steps (a) and (b) means that different amines and either organohaloformates or organochloroformates may be added to each reaction zone in the library forming process, if desired. In one embodiment of the process of the invention, each combination of amine and either organohaloformate or organochloroformate added to each library reaction zone (e.g., wells of a wellplate) is different. Thus, the same amine may be added to each row of a wellplate apparatus (as per Fig. 1) and the same aldehyde reactant may be added to the same column of a wellplate apparatus to give a different combination of reactants in each well (viz., reaction zone) that will expectantly yield a different library compound. Alternatively, where it is desirable to have replicate samples, the same combination of amine and either organohaloformate or organochloroformate may be added to different reaction zones.

Detail of Operation for the Carbamate Library Process Step (a):

The carbamate library making process is conducted using in step (a) primary and/or secondary amines which are the same as described for step (a) of the Amide Library Process, supra., the disclosure of which is incorporated herein by reference.

Detail of Operation for the Carbamate Library Process - Step (b):

The organohaloformate reactant in step (b) of the carbamate library making process of the invention is represented by the formula,

$$R_3OC(O)X$$

where X is halo and R3 is a non-interfering substituent selected from non-interfering radical selected from Suitable non-interfering radicals are $C_1$-$C_{10}$ alkyl, $C_2$-$C_{10}$ alkenyl, $C_2$-$C_{10}$ alkynyl, $C_1$-$C_{10}$ alkoxy, $C_7$-$C_{12}$ aralkyl, $C_7$-$C_{12}$ alkaryl, $C_3$-$C_{10}$ cycloalkyl, $C_3$-$C_{10}$ cycloalkenyl, phenyl, substituted phenyl, toluyl, xylenyl, biphenyl, $C_2$-$C_{12}$ alkoxyalkyl, $C_1$-$C_6$ alkylsulfinyl, $C_1$-$C_{10}$ alkylsulfonyl,-$(CH_2)_m$-0-(aryl), aryl, substituted aryl, amidino, fluoroalkyl, aryloxyalkyl, heterocyclic radical, substituted heterocyclic radical, and nitroalkyl; where m is from 1 to 8. Preferred non-interfering radicals are $C_1$-$C_{10}$ alkyl, $C_2$-$C_{10}$ alkenyl, $C_1$-$C_{10}$ alkoxy, $C_7$-$C_{12}$ aralkyl, $C_7$-$C_{12}$ alkaryl, $C_3$-$C_{10}$ cycloalkyl, $C_3$-$C_{10}$ cycloalkenyl, phenyl, aryl, and substituted aryl.

Particularly preferred are organolhaloformates where the halo group is chloro and the organo group is alkyl.

Preferred organohaloformates are aliphatic, aromatic and heterocyclic haloformates having a molecular weight of from 94 to 600.

Illustrative organohaloformate compounds suitable for use in the carbamate library making process of the invention are as follows:

Organohaloformates --

9-fluorenylmethyl chloroformate
phenyl chloroformate
4-chlorophenyl chloroformate
methyl chloroformate
benzyl chloroformate
vinyl chloroformate
isobutyl chloroformate
2-ethylhexyl chloroformate
ethyl chloroformate
2-bromoethyl chloroformate
2-chloroethyl chloroformate
1-chloroethyl chloroformate
allyl chloroformate
n-propyl chloroformate
butyl chloroformate
n-hexyl chloroformate
octyl chloroformate
2,2,2-trichloro-1,1-dimethylethyl chloroformate
2,2,2-trichloroethyl chloroformate
cholesteryl chloroformate
4-nitrophenyl chloroformate
4-nitrobenzyl chloroformate
(-)-menthyl chloroformate
4-t-butylcyclohexyl chloroformate
cetyl chloroformate
(+)-1-(9-fluorenyl)ethyl chloroformate
isopropyl chloroformate
3-chlorocyclohexyl chloroformate
decyl chloroformate
oleyl chloroformate

octadecyl chloroformate
butenediol bischloroformate
2-chlorobenzyl chloroformate
4-chlorobutyl chloroformate
(+)-menthyl chloroformate
4,5-dimethoxy-2-nitrobenzyl chloroformate
cyclopentyl chloroformate
t-butylcyclohexyl chloroformate
menthylchloroformate
p-tolyl chloroformate
4-bromophenyl chloroformate
4-fluorophenyl chloroformate
4-methoxyphenyl chloroformate
2-nitrophenyl chloroformate
4-methoxycarbonylphenyl chloroformate
1-chloro-2-methylpropyl chloroformate
(+/-)-1,2,2,2-tetrachloroethyl chloroformate
2 ,2-dichloroethyl chloroformate
myristyl chloroformate
cyclohexyl chloroformate
chloromethyl chloroformate.

In step (b) a second reagent addition (iii) of neutralizing agent is used to neutralize the acid, HX, resulting from the reaction of the acyl halide and the amine reactant of step (a). Conventional neutralizing agents such as alkali and alkaline-earth hydroxides may be used. However, it is preferred to use a resin bound base such as polyvinyl pyridine as a solid phase scavenger of HX formed in the course of the reaction. Resin bound (or other solid) neutralizing agents may be conveniently removed from the reaction in the separation step (d), infra. The amount of acid, HX, anticipated to be formed may be determined by reference to Scheme 2, infra, namely, 1 mole of HX formed per mole of amine reagent that reacts.

Detail of Operation for the Carbamate Library Process - Step (c):

The solid-supported scavenger is used in the carbamate forming process of the invention to remove excess soluble organohaloformate reagent. The solid state scavenger is represented by the formula:

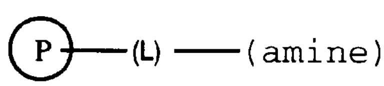

The scavenger used in this carbamate combinatorial process is the same as that described in the prior section entitled, "Detail of Operation for the Amide Library Process - Step (c)", supra., the disclosure of which is incorporated herein by reference;, except that any mention of acyl halide in that section is equivalent to the use of organohaloformate in this section.

Detail of Operation for the Carbamate Library Process - Step (d):

The final step of the carbamate library making process is separation of the scavenger from the reaction zone. This step is the same as that described in the section, entitled, "Detail of Operation for the Amide Library Process - Step (d)" supra., the disclosure of which is incorporated herein by reference; except that any mention of acyl halide in that section is equivalent to the use of organohaloformate in this section.

Other details of the Carbamate Process:

The details of requirements for reaction medium, reaction zone, proportions of reactants, reaction conditions, enpoint determination, sequence of addition of reactants for the carbamate combinatorial process is the same as that described in the section, entitled, "Other Details of the Amide Library making process", supra., the disclosure of which is incorporated herein by reference; except that any mention of acyl halide in that section is equivalent to the use of

organohaloformate in this section.

Carbamate Library Process - Reaction Scheme:

An illustrative reaction scheme for the formation of carbamate libraries is given in Scheme 2, as follows:

Scheme 2

USE OF AMINOMETHYLATED POLYMER
IN THE PURIFICATION OF CARBAMATES

STEP 1 - Synthesis of Carbamate

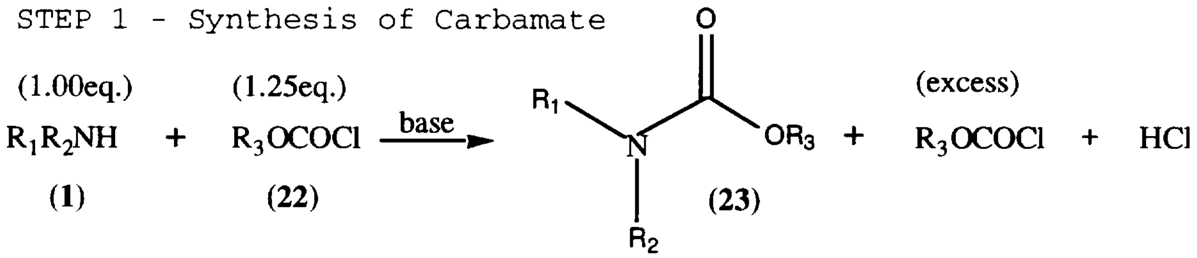


(1) (22) (23)

STEP 2 - Removal of excess $R_3OCOCl$

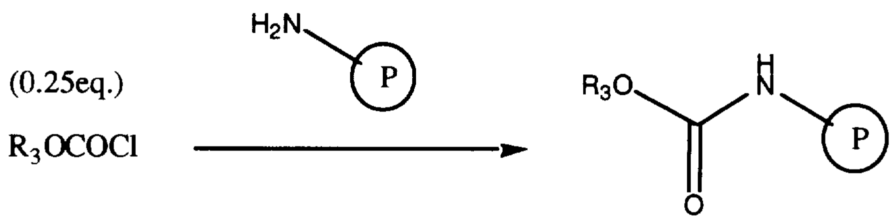


(4) (25)

Preparation of Carbamates:

In Step 1 a primary or secondary amine (1) is combined in a non-nucleophilic solvent such as ethanol-free chloroform with an excess of alkyl chloroformate (22) and a stoichiometric excess of neutralizing agent (viz., solid supported dimethylaminopyridine or piperidine-N-polystyrene to neutralize HCl released in the reaction) to form a carbamate product (23) contaminated with alkylchloroformate.

In Step 2 there is added to the reaction product of Step 1 a stoichiometric excess of solid supported scavenger (4) in the form of aminomethylated polymer (aminomethylated polystyrene, available from Aldrich Chemical Co.). This scavenger resin-bound primary amine reacts to form a covalent bond between the resin bound amine functionality and the alkyl chloroformate, yielding a resin bound carbamate (25).

In a final step (not illustrated) the product carbamate and scavenger in chloroform medium are filtered through a cotton plug. The resin retained by the plug is rinsed free of product by washing with additional chloroform. A purified carbamate product is retained (in solution) and may be isolated free of reaction medium by solvent evaporation.

VI. Sulfonamide combinatorial library - General Description:

The sulfonamide library of the invention is a combinatorial library having library compounds represented by the formula (L):

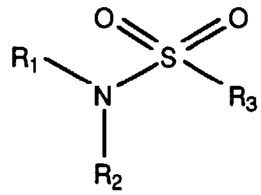

(L)

where $R_1$, $R_2$, and $R_3$ are defined in the following sections.

A preferred sulfonamide library is formed from diverse amine and diverse organolsulfonylhalide reactants.

The following sections VII and VIII describe a solution phase process for making sulfonamide combinatorial libraries.

VII. Description of the scavenger assisted solution phase process for making sulfonamide combinatorial libraries of the invention.

This invention is a scavenger assisted combinatoric process for preparing a library of compounds having a sulfonamide scaffold with three variable substituents, said compounds represented by the formula (L);

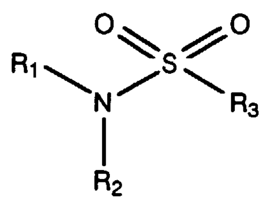

(L)

said process comprising the steps of:

a) adding to each reaction zone at least (n) equivalents of a solvent soluble primary or secondary amine reactant represented by the formula:

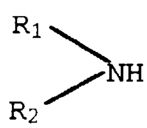

where $R_1$ and $R_2$ are independently selected from hydrogen and non-interfering substituents with the proviso that $R_1$ and $R_2$ are not both hydrogen;

b) adding to each reaction zone of step (a) containing a liquid medium;

(i) at least 1.1(n) equivalents of an organosulfonylhalide represented by the formula;

$$R_3SO_2X$$

where X is halo and $R_3$ is a non-interfering substituent, and;

ii) a base in an amount sufficient to neutralize the acid, HX, formed;

c) adding to each reaction zone of step (b) a solid supported amine functional scavenger represented by the formula;

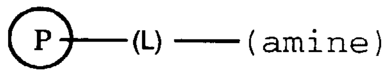

wherein;

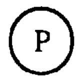

is a solid-support insoluble in the liquid medium of the reaction zone, and -(L)- is a divalent linking group, (amine) is either a primary or secondary amine substituent; and adding said scavenger in an amount at least equal to the excess equivalents of unreacted organosulfonylhalide reactant used in step (b), and maintaining said reaction zone at a temperature and for a time sufficient to permit reaction of said excess alkyl sulfonyl halide reactant and said scavenger;

d) separating the solid supported scavenger from each reaction zone of step (c) and recovering each substantially purified sulfonamide library compound.

The "adding to each reaction zone" requirement for the amine and organosulfonylhalide reactant in steps (a) and (b) means that different amines and organosulfonylhalides may be added to each reaction zone in the library forming process, if desired. In one embodiment of the process of the invention, each combination of amine and organosulfonylhalides added to each library reaction zone (e.g., wells of a wellplate) is different. Thus, the same amine may be added to each row of a wellplate apparatus (as per Fig. 1) and the same aldehyde reactant may be added to the same column of a wellplate apparatus to give a different combination of reactants in each well (viz., reaction zone) that will expectantly yield a different library compound. Alternatively, where it is desirable to have replicate samples, the same combination of amine and organosulfonylhalide may be added to different reaction zones.

Detail of Operation of the Sulfonamide Library Process - Step (a):

The sulfonamide library making process is conducted using in step (a) primary and/or secondary amines which are the same as those described in the prior section entitled, "Detail of Operation for the Amide Library Process - Step (a)", supra., the disclosure of which is incorporated herein by reference.

Detail of Operation of the Sulfonamide Library Process Step (b):

The organosulfonylhalide reactant is represented by the formula;

$$R_3SO_2X$$

where X is halo and $R_3$ is a non-interfering substituent; selected from $C_1$-$C_{10}$ alkyl, $C_2$-$C_{10}$ alkenyl, $C_2$-$C_{10}$ alkynyl, $C_1$-$C_{10}$ alkoxy, $C_7$-$C_{12}$ aralkyl, $C_7$-$C_{12}$ alkaryl, $C_3$-$C_{10}$ cycloalkyl, $C_3$-$C_{10}$ cycloalkenyl, phenyl, substituted phenyl, toluyl, xylenyl, biphenyl, $C_2$-$C_{12}$ alkoxyalkyl, $C_1$-$C_6$ alkylsulfinyl, $C_1$-$C_{10}$ alkylsulfonyl, $-(CH_2)_m$-0-(aryl), aryl, substituted aryl, amidino, fluoroalkyl, aryloxyalkyl, heterocyclic radical, substituted heterocyclic radical, and nitroalkyl; where m is from 1 to 8. Preferred non-interfering radicals are $C_1$-$C_{10}$ alkyl, $C_2$-$C_{10}$ alkenyl, $C_1$-$C_{10}$ alkoxy, $C_7$-$C_{12}$ aralkyl, $C_7$-$C_{12}$ alkaryl, $C_3$-$C_{10}$ cycloalkyl, $C_3$-$C_{10}$ cycloalkenyl, phenyl, aryl, and substituted aryl.

Preferred organosulfonylhalides are aliphatic, aromatic, or heterocyclic organosulfonylhalides having a molecular weight from 114 to 600.

Particularly preferred are organosulfonylhalide where the halo group is chloro and the organo group is alkyl. Illustrative organosulfonylhalide halides compounds suitable for use in the sulfonamide library making process of the invention are as follows:

Organosulfonylhalides --

1-naphthalenesulfonyl chloride
dansyl chloride

2-naphthalenesulfonyl chloride
2-acetamido-4-methyl-5-thiazolesulfonyl chloride
2-thiophenesulfonyl chloride
8-quinolinesulfonyl chloride
benzenesulfonyl chloride
pentafluorobenzenesulfonyl chloride
2,5-dichlorobenzenesulfonyl chloride
2-nitrobenzenesulfonyl chloride
2,4-dinitrobenzenesulfonyl chloride
3,5-dichloro-2-hydroxybenzenesulfonyl chloride
2,4,6-triisopropylbenzenesulfonyl chloride
2-mesitylenesulfonyl chloride
3-nitrobenzenesulfonyl chloride
p-bromobenzenesulfonyl chloride
4-fluorobenzenesulfonyl chloride
4-chlorobenzenesulfonyl chloride
4-chloro-3-nitrobenzenesulfonyl chloride
pipsyl chloride
4-nitrobenzenesulfonyl chloride
4-methoxybenzenesulfonyl chloride
4-tert-butylbenzenesulfonyl chloride
p-toluenesulfonyl chloride
trifluoromethanesulfonyl chloride
trichloromethanesulfonyl chloride
isopropylsulfonyl chloride
methanesulfonyl chloride
alpha-toluenesulfonyl chloride
trans-beta-styrenesulfonyl chloride
2,2,2-trifluoroethanesulfonyl chloride
1-hexadecanesulfonyl chloride
ethanesulfonyl chloride
2-chloroethanesulfonyl chloride
1-propanesulfonyl chloride
3-chloropropanesulfonyl chloride
1-butanesulfonyl chloride
methyl 2-(chlorosulfonyl)benzoate
2-nitro-4-(trifluoromethyl)benzenesulfonyl chloride
3-(trifluoromethyl)benzenesulfonyl chloride
1-octanesulfonyl chloride
4-(trifluoromethoxy)benzenesulphonyl chloride
(1r)-(-)-10-camphorsulfonyl chloride
d-(+)-10-camphorsulfonyl chloride
(+/-)-10-camphorsulfonyl chloride
2-nitro-alpha-toluenesulfonyl chloride.

Suitable sulfonyl halides for the practice of the process of the invention are those represented by the formulae:

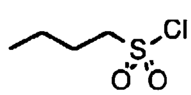

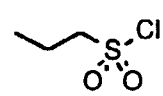

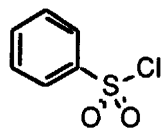

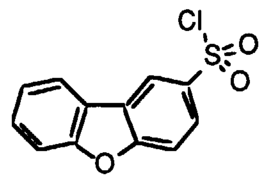

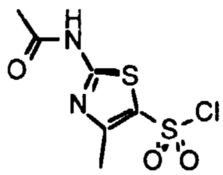

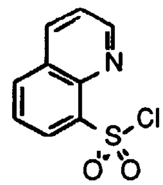

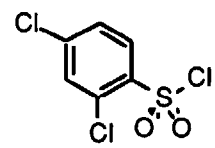

and

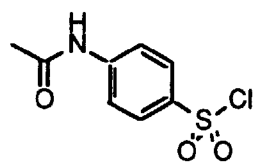

.

In step (b) a second reagent addition (ii) of neutralizing agent is used to neutralize the acid, HX, resulting from the reaction of the acyl halide and the amine reactant of step (a). Conventional neutralizing agents such as alkali and alkaline-earth hydroxides may be used. However, it is preferred to use a resin bound base such as piperidinomethylpolystyrene as a solid phase scavenger of HX formed in the course of the reaction. Resin bound (or other solid neutralizing agents) may be conveniently removed from the reaction in the separation step (d), infra. The amount of acid, HX, anticipated to be formed may be determined by reference to Scheme 3, infra, namely, 1 mole of HX formed per mole amine reactant that reacted.

<u>Detail of Operation of the Sulfonamide Library Process - Step (c):</u>

The solid-supported scavenger is used in the sulfonamide forming process of the invention to remove excess soluble organosulfonylhalide reagent. The solid state scavenger is represented by the formula:

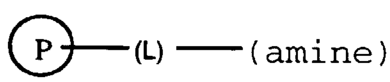


The scavenger used in this sulfonamide combinatorial process is the same as that described in the prior section entitled, "Detail of Operation for the Amide Library Process - Step (c)", supra., the disclosure of which is incorporated herein by reference; except that any mention of acyl halide in that section is equivalent to the use of organosulfonylhalide in this section.

Detail of Operation for the Sulfonamide Library Process Step (d):

The final step of the sulfonamide library making process is separation of the scavenger from the reaction zone. This step is the same as that described in the section, entitled, "Detail of Operation for the Amide Library Process - Step (d)" supra., the disclosure of which is incorporated herein by reference; except that any mention of acyl halide in that section is equivalent to the use of organosulfonylhalide in this section.

Other details of the Sulfonamide Process:

The details of requirements for reaction medium, reaction zone, proportions of reactants, reaction conditions, enpoint determination, sequence of addition of reactants for the sulfonamide combinatorial process is the same as that described in the section, entitled, "Other Details of the Amide Library making process", supra., the disclosure of which is incorporated herein by reference; except that any mention of acyl halide in that section is equivalent to the use of organosulfonylhalide in this section.

Sulfonamide Library Process - Reaction Scheme:

An illustrative reaction scheme for the formation of sulfonamide libraries is given in Scheme 3, as follows:

Scheme 3

USE OF AMINOMETHYLATED POLYMER
IN THE PURIFICATION OF SULFONAMIDES

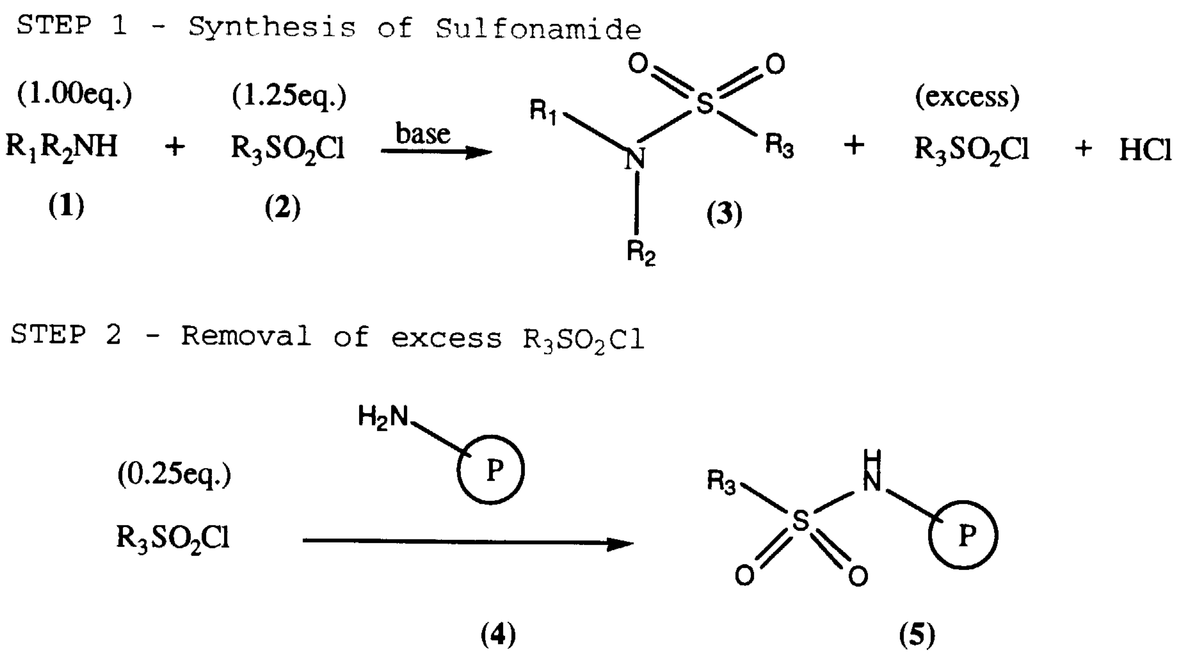


Preparation of Sulfonamides:

In Step 1 a primary or secondary amine (1) is combined in a non-nucleophilic solvent such as ethanol-free chloroform with an excess of sulfonyl chloride (2) and a stoichiometric excess of neutralizing agent (viz., solid supported dimethylaminopyridine or piperidinomethylpolystyrene to neutralize HCl released in the reaction) to form a sulfonamide product (3) contaminated with sulfonyl chloride.

In Step 2 there is added to the reaction product of Step 1 a stoichiometric excess of solid supported scavenger

(4) in the form of aminomethylated polymer (aminomethylated polystyrene, available from Aldrich Chemical Co.). This scavenger resin-bound primary amine reacts to form a covalent bond between the resin bound amine functionality and the sulfonyl chloride, yielding a resin bound sulfonamide (5).

In a final step (not illustrated) the product sulfonamide and scavenger in chloroform medium are filtered through a cotton plug. The resin retained by the plug is rinsed free of product by washing with additional chloroform. A purified sulfonamide product is retained (in solution) and may be isolated free of reaction medium by solvent evaporation.

VIII. General Reaction Scheme - for making amide, carbamate, and sulfonamide combinatorial libraries:

The relationship of the amide, carbamate, and sulfonamide processes of the invention is depicted in the combined process Scheme 4, shown below:

Scheme 4

USE OF AMINOMETHYLATED POLYSTYRENE IN THE PURIFICATION OF AMIDES, CARBAMATES, AND SULFONAMIDES

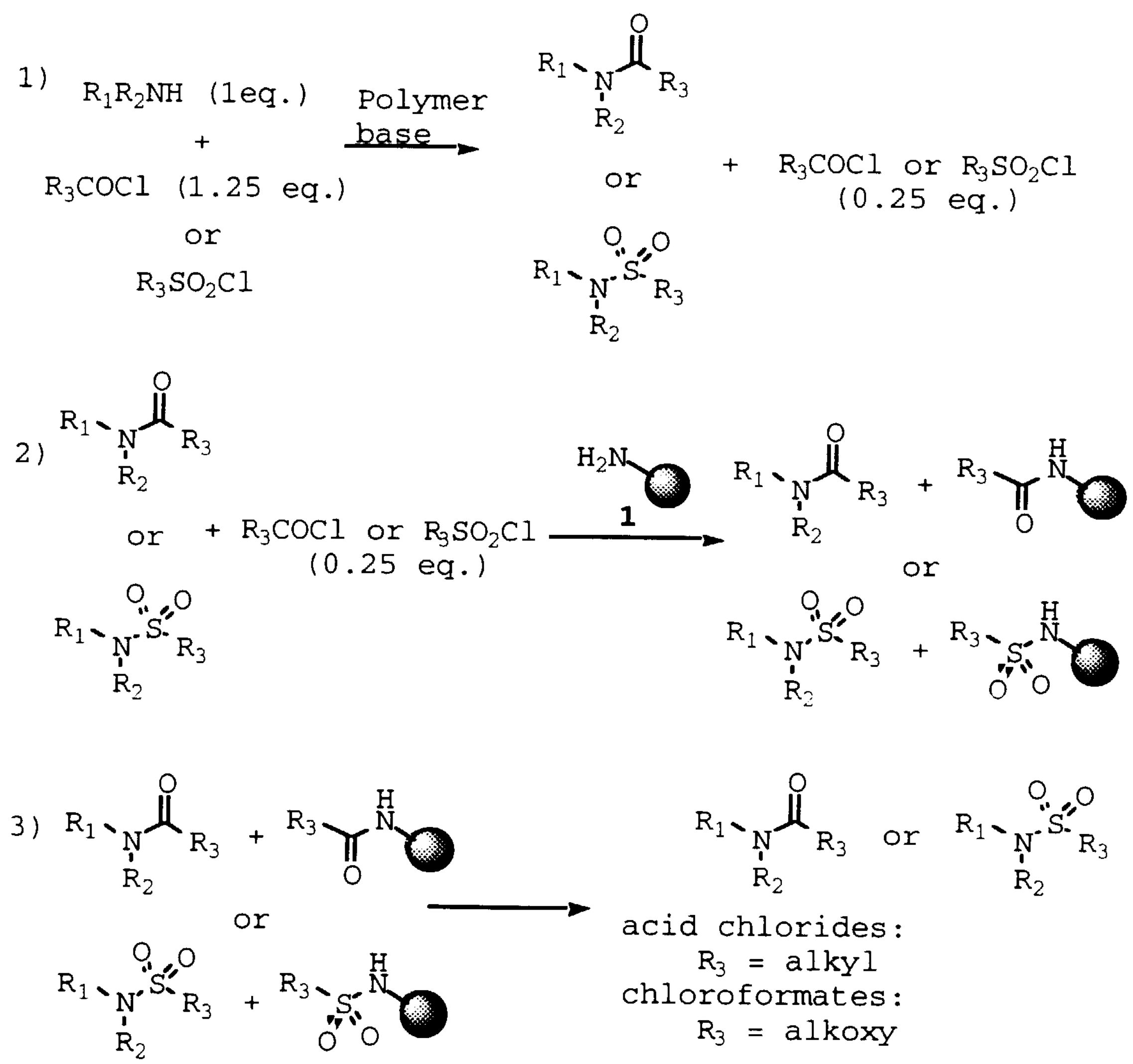


In the use of aminomethylated polystyrene **1** as a scavenger for acid chlorides, chloroformates or sulfonyl chlorides in the synthesis of amides, carbamates, or sulfonamides (above), a primary or secondary amine is combined in a non-nucleophilic solvent such as ethanol-free chloroform with an excess of either an acid chloride, chloroformate, or sulfonyl chloride and an excess of solid-supported dimethylaminopyridine (available from Reilly Scientific, added to neutralize

HCl released in the reaction) or piperidinomethylpolystyrene (prepared in house; also available from Fluka) to form an amide, carbamate, or sulfonamide as product, contaminated with starting acid chloride, chloroformate, or sulfonyl chloride (Step 1). To this mixture is added an excess of aminomethylated polystyrene, available from Aldrich Chemicals. This resin-bound primary amine reacts to form a covalent bond between the resin-bound amine and the acid chloride, chloroformate, or sulfonyl chloride, yielding a resin-bound amide, carbamate, or sulfonamide (Step 2). In the final step, the mixture of product amide, carbamate or sulfonamide and resin in chloroform is filtered through a cotton plug and the resin, retained by the plug, is rinsed free of any product by washing with additional solvent (Step 3).

Other Utilities - Amides:

Derivatives of amides (capable of being prepared by the method of this invention) have utility in a number of technological areas, as follows:
A) Fatty acid amides are used as antislip and antiblock additives for polyethylene film, water repellants for textiles, mold release agents, ink additives, lubricant additives, and etc. Useful fatty acid amids include octadecanamide, 13-docosenamide, polyoxyethylated octadecenamide and N,N-dimethyl-dedecanamide, and their homologs and analogs. Useful fatty acid amids include octadecanamide, 13-docosenamide, polyoxyethylated octadecenamide and N,N-dimethyl-dedecanamide, and their homologs and analogs.

Other Utilities - Carbamates:

Derivatives of carbamates (capable of being prepared by the method of this invention) have utility in a number of technological areas, as follows:
A) Carbamate insecticidal activity for a wide variety of structures, such as carbaryl, carbofuran, propoxur, dioxacarb, and bendiocarb. 1-napthtyl N-methylcarbamate is a broad spectrum insecticide. 2-isoropoxyphenyl N-methylcarbamate is used for the control of mosquitoes.

Other Utilities - Sulfonamides:

Derivatives of sulfonamides (capable of being prepared by the method of this invention) have utility in a number of technological areas, as follows:
A) N-alkyl substituted aromatic sulfonamides are useful as dye assistants - US Pat. No. 5,437,690.

IX. Wellplate Apparatus containing library compounds prepared by the process of the invention:

The processes of making the amide, carbamate, and sulfonamide libraries of the invention may be conveniently carried out in a wellplate apparatus such as illustrated in Fig. 1 and Fig. 2, hereinafter described. It is particularly advantageous to carry out the method of the invention in a standard wellplate apparatus such as a plastic 96 well microtiter plate.

Typically the wellplate apparatus is in the form of a rigid or semi-rigid plate, said plate having a common surface containing openings of a plurality of vessels arranged in rows and columns. A standard form of wellplate apparatus is a rectangular plastic plate having 8 rows and 12 columns (total 96) of liquid retaining depressions on its surface. A wellplate apparatus may optionally have other elements of structure such as a top or cover (e.g., plastic or foil), a bottom in a form such as a plate or reservoir, clamping means to secure the wellplate and prevent loss of its contained compounds.

The sequence of operations to be used for library generation with the wellplate is as follows:

The wellplate apparatus of the invention:

A wellplate inoculated with the novel amide or carbamate or sulfonamide library compounds of the invention is itself a new construct or apparatus which has particular utility in an assay kit used to discover lead compounds. A suitable system of operation and related apparatus are made as follows:

1. Reaction zones are made by drilling 96 holes in the bottom of 96 deepwell titer plates and putting a porous frit in the bottom of each well.
2. The plate is put in a clamp assembly to seal the bottom of the wells.
3. Synthesis is begun by adding reagents to their assigned plate coordinates (reaction zone).
4. The plate is capped then tumbled to mix the reagents.
5. Solid supported scavenger is added to each reaction zone after completion of the reaction is shown by thin layer

chromatography.

6. After sufficient reaction time the plate is removed from the clamp and the resin washed.
7. The solution containing product is filtered and the solution collected by transfer into another 96 well plate.
8. The reaction products (library compounds) are analyzed by thin layer chromatography.

Detailed Description of the Drawings

FIG. 1 illustrates the top surface of a wellplate apparatus of the invention. The wellplate (3) is a plastic plate with 96 wells (depressions) capable of holding liquids. When used in the parallel array synthesis individual reaction products are prepared in each well and are labeled by the wellplate coordinates. The shaded circles in the Figure represent wells filled with amide, or 3 carbamate, or sulfonamide library compounds prepared by the solution phase combinatorial processes of the invention. The library compound at location (1), for example, is identified by the alphanumeric coordinate, "A6."

FIG. 2 illustrates a side view of a wellplate apparatus used in the Assay Kit of the invention. The wellplate (5) contains wells (7) with a filter (9) and liquid reaction medium containing scavenger (11). The wells have an outlet at bottom which is sealed by gasket (13) held in place by top cover (15) and bottom cover (17) maintained in position by clamp (19).

X. Assay Kits using wellplates with the library compounds of the invention:

This invention includes an assay kit for identification of pharmaceutical lead compounds. The assay kit comprises as essential parts, (i) wellplate apparatus (containing in its wells the tertiary amine library compounds of the invention), and (ii) biological assay materials.

The wellplate apparatus in the kit may comprise a set of wellplate apparatus such as illustrated in Fig. 1. The library compounds contained in each wellplate may be prepared by either the amide, carbamate, or sulfonamide combinatorial processes taught herein. Preferably the wellplate apparatus has the form of a standard 96 well microtiter plate.

The assay kit also contains biological assay materials These biological assay materials are generally in vitro tests known to be predictive of success for an associated disease state. Illustrative of biological assay materials useful in the kit of this invention are those required to conduct the following assays:

In vitro assays:
- Enzymatic Inhibition
- Receptor-ligand binding
- Protein-protein Interaction
- Protein-DNA Interaction

Cell-based, Functional assays:
- Transcriptional Regulation
- Signal Transduction/ Second Messenger
- Viral Infectivity

Add, Incubate, & Read assays:
- Scintillation Proximity Assays
- Angiotensin II SPA receptor binding assay
- Endothelin converting enzyme[$^{125}$I] SPA assay
- HIV proteinase [$^{125}$I] SPA enzyme assay
- Cholesteryl ester transfer protein (CETP) [$^{3}$H] SPA assay

Fluorescence Polarization Assays
Fluorescence Correlation Spectroscopy
Colorimetric Biosensors
$Ca^{2+}$-EGTA Dyes for Cell-based assays
Reporter Gene Constructs for cell based assays
- Luciferase, green fluorescent protein,
- β-lactamase

Electrical cell impedance sensor assays

EXAMPLES

Procedures for the Synthesis of Polymer Bound Reagents

Piperidinomethylpolysterene: (Note: this resin is also commercially available with reduced loading capacity from

Fluka) A solution of 50 g (215 mmol, 1 equiv.) of chloromethyl polystyrene (Fluka, 200-400 mesh, 2% DVB, 4.3 mmol/g), 6.25 g (45 mmol, 0.2 equiv.) of $K_2CO_3$ and 65 mL (888 mmol, 4.1 equiv) of piperidine in 500 mL of anhydrous DMF was heated overnight at 90_C. The resin was filtered, washed with DMF (500 mL) and water (500 mL) and then stirred for 20 min. in 400 mL of water. After filtration the resin was washed with water and dioxane and then stirred for 20 min. in 400 mL of ethanol. The resin was filtered again and then sequentially washed with ethanol, THF and ether. After overnight drying at 55_C in a vacuum oven a light yellow resin was obtained. Elemental Analysis: C, 85.73; H, 8.95; N, 5.28 (3.8 mmol/g loading); Cl, None. Commercially available resins:
Aminomethylated polystyrene, Aldrich Chemicals
Poly-DMAP and Polyvinylpyridine, Reilly Scientific

## Claims

1. A scavenger assisted solution phase combinatorial process for preparing a library of compounds having an amide scaffold with three variable substituents, wherein each library compound is made in a separate reaction zone and is represented by the formula (I);

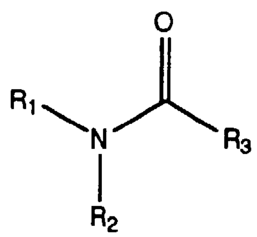

(I)

said process comprising the steps of:

a) adding to each reaction zone containing a liquid medium (n) equivalents of a solvent soluble primary or secondary amine reactant represented by the formula:

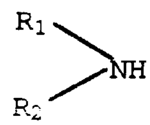

where $R_1$ and $R_2$ are independently selected from hydrogen and non-interfering substituents with the proviso that $R_1$ and $R_2$ are not both hydrogen;

b) adding to each reaction zone of step (a) containing a liquid medium;

(i) at least 1.1(n) equivalents of an acyl halide represented by the formula;

$$R_3C(O)X$$

where X is halo and $R_3$ is a non-interfering substituent, and;

ii) a base in an amount sufficient to neutralize the acid, HX, formed;

c) adding to each reaction zone of step (b) a solid supported amine functional scavenger represented by the formula;

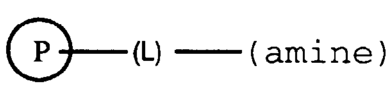

wherein;

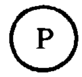

is a solid-support insoluble in the liquid medium of the reaction zone, and -(L)- is a divalent linking group, (amine) is either a primary or secondary amine substituent; and adding said scavenger in an amount at least equal to the excess equivalents of unreacted acyl halide reactant used in step (b), and maintaining said reaction zone at a temperature and for a time sufficient to permit reaction of said excess acyl halide reactant and said scavenger;

d) separating the solid supported scavenger from each reaction zone of step (c) and recovering each substantially purified amide library compound.

**2.** The library of amide compounds prepared by the process of claim 1.

**3.** An assay kit for identification of pharmaceutical lead compounds, comprising biological assay materials and wellplate apparatus;
wherein the improvement comprises using as wellplate apparatus a wellplate containing in each well library compound of a diverse combinatorial amide library prepared by the processes of claim 1.

**4.** Wellplate apparatus suitable as a replaceable element in an automated assay machine wherein the improvement comprises;
using as the wellplate apparatus a diverse amide combinatorial wellplate, wherein each well independently contains an amide library compound prepared by the process of claim 1.

**5.** A scavenger assisted solution phase combinatorial process for preparing a library of compounds having a carbamate scaffold with three variable substituents, wherein each library compound is made in a separate reaction zone and is represented by the formula (V);

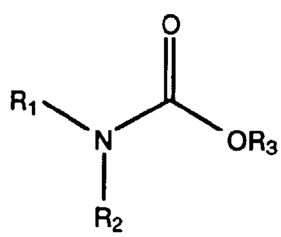 (V)

said process comprising the steps of:

a) adding to each reaction zone at least (n) equivalents of a solvent soluble primary or secondary amine reactant represented by the formula:

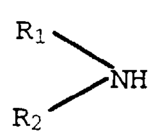

where $R_1$ and $R_2$ are independently selected from hydrogen and non-interfering substituents, provided $R_1$ and $R_2$ are not both hydrogen;

b) adding to each reaction zone of step (a);

(i) at least 1.1(n) equivalents of an organohaloformate, represented by the formula,

$$R_3OC(O)X$$

where X is halogen and $R_3$ is a non-interfering substituent; or,

(ii) at least 1.1(n) equivalents of an organocyanoformate, represented by the formula,

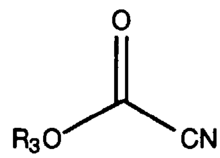

where $R_3$ is a non-interfering substituent; and

(iii) adding to each reaction zone a base in an amount sufficient to neutralize the acid, HX, formed;

c) adding to each reaction zone of step (b) a solid supported amine functional scavenger represented by the formula;

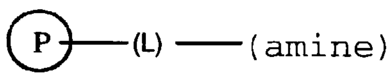

wherein;

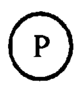

is a solid-support insoluble in the liquid medium of the reaction zone, and -(L)- is a divalent linking group, (amine) is either a primary or secondary amine substituent; and adding said scavenger in an amount at least equal to the excess equivalents of unreacted organohaloformate reactant used in step (b), and maintaining said reaction zone at a temperature and for a time sufficient to permit reaction of said excess organohaloformate reactant and said scavenger;

d) separating the solid supported scavenger from each reaction zone of step (c) and recovering each substantially purified carbamate library compound.

**6.** The library of carbamate compounds prepared by the process of claim 5.

**7.** The individual carbamate library compounds in the carbamate library of claim 6.

**8.** Wellplate apparatus suitable as a replaceable element in an automated assay machine wherein the improvement comprises, using as the wellplate apparatus a diverse carbamate combinatorial wellplate, wherein each well independently contains carbamate library compound prepared by the process of claim 5.

**9.** A scavenger assisted solution phase combinatorial process for preparing a library of compounds having a sulfonamide scaffold with three variable substituents, wherein each library compound is made in a separate reaction zone and is represented by the formula (L);

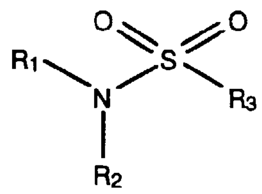

(L)

said process comprising the steps of:

a) adding to each reaction zone at least (n) equivalents of a solvent soluble primary or secondary amine reactant represented by the formula:

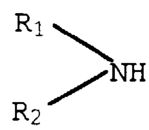

where $R_1$ and $R_2$ are independently selected from hydrogen and non-interfering substituents with the proviso that $R_1$ and $R_2$ are not both hydrogen;

b) adding to each reaction zone of step (a) containing a liquid medium;

(i) at least 1.1(n) equivalents of an organosulfonylhalide represented by the formula;

$$R_3SO_2X$$

where X is halo and $R_3$ is a non-interfering substituent, and;

ii) a base in an amount sufficient to neutralize the acid, HX, formed;

c) adding to each reaction zone of step (b) a solid supported amine functional scavenger represented by the formula;

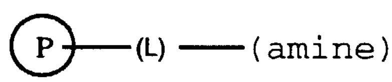

wherein;

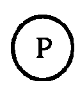

is a solid-support insoluble in the liquid medium of the reaction zone, and -(L)- is a divalent linking group, (amine) is either a primary or secondary amine substituent; and adding said scavenger in an amount at least equal to the excess equivalents of unreacted organosulfonylhalide reactant used in step (b), and maintaining said reaction zone at a temperature and for a time sufficient to permit reaction of said excess alkyl sulfonyl halide reactant and said scavenger;

d) separating the solid supported scavenger from each reaction zone of step (c) and recovering each substantially purified sulfonamide library compound.

**10.** The library of sulfonamide compounds prepared by the process of claim 9.

**11.** An assay kit for identification of pharmaceutical lead compounds, comprising biological assay materials and wellplate apparatus;
wherein the improvement comprises.using as wellplate apparatus a wellplate containing in each well library compound of a diverse combinatorial sulfonamide library prepared by the processes of claims 9.

**12.** Wellplate apparatus suitable as a replaceable element in an automated assay machine wherein the improvement comprises,
using as the wellplate apparatus a diverse sulfonamide combinatorial wellplate, wherein each well independently contains an sulfonamide library compound prepared by the process of claim 9.

FIG.1

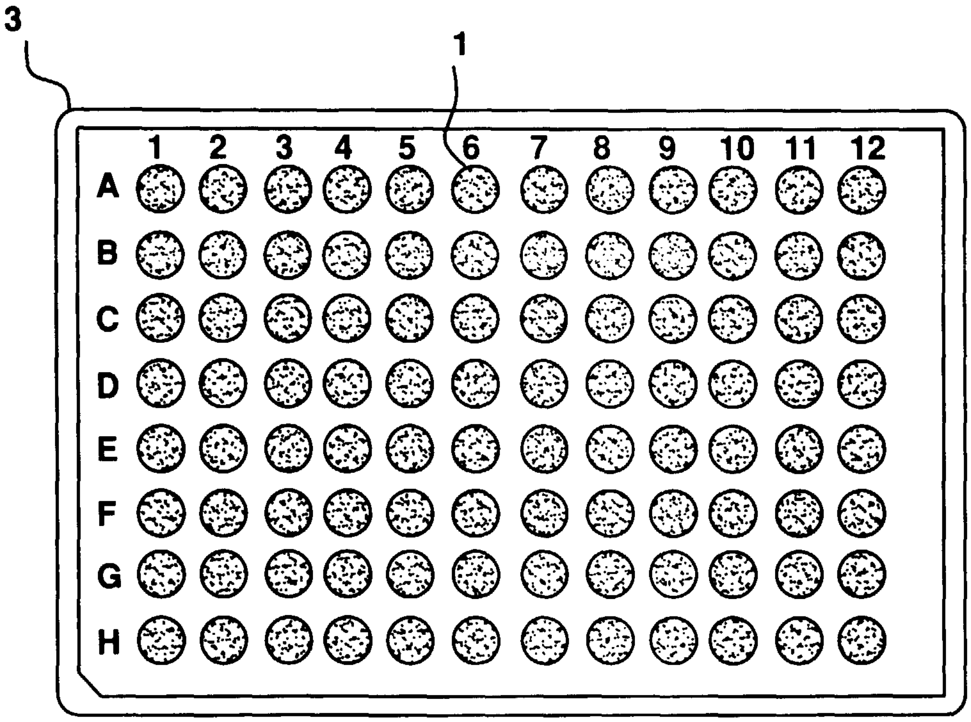

FIG.2